# EUROPEAN PATENT APPLICATION

(11) **EP 4 600 256 A1**
(43) Date of publication of application: **13.08.2025**
(21) Application number: 24810137.0
(22) Date of filing: 24.04.2024
(51) Int. Cl.: C07K 14/135, A61K 39/155, A61K 9/51, A61P 31/14, C12N 15/45

(54) **RESPIRATORY SYNCYTIAL VIRUS VACCINE AS WELL AS PREPARATION METHOD THEREFOR AND USE THEREOF**

(30) Priority: 19.05.2023 CN 202310570847
(71) Applicant: Liverna Therapeutics Inc., Hengqin Zhuhai, Guangdong 519031 (CN)
(72) Inventor: LI, Jianglong, Zhuhai, Guangdong 519031 (CN); PENG, Yucai, Zhuhai, Guangdong 519031 (CN)
(74) Representative: Rentsch Partner AG
(86) International application number: PCT/CN2024/089467
(87) International publication number: WO 2024/239890

(57) **Abstract**

The present disclosure relates to an immunological composition comprising or encoding a human respiratory syncytial virus antigen, and the immunological composition is selected from a nucleic acid immunological composition, a polypeptide immunological composition or a viral immunological composition.

## Description

This application claims the priority of the Chinese patent application filed on May 19, 2023 (Application No.: 202310570847.2, Invention Title: Respiratory Syncytial Virus Vaccine as well as Preparation Method therefor and Use thereof), the entire contents of which are incorporated by reference into this application in its entirety.

### Technical Field

The present disclosure relates to the technical field of genetic medicine, and in particular to a respiratory syncytial virus vaccine, a method of prepartion thereof and uses thereof.

### Background Art

The statement herein merely provides background information related to the present disclosure and may not necessarily constitute the prior art.

Respiratory syncytial virus (RSV) is the most common pathogen of bronchiolitis and pneumonia in infants and young children, which seriously endangers their health. The infection with RSV does not provide lasting immunity, so repeated infection is very common. RSV infection is global and can cause local outbreaks, and has become a global public health concern. However, there are currently no approved RSV vaccines.

The RSV genome is about 15 to 16 kb in size and encodes 11 proteins, including 8 structural proteins and 3 non-structural proteins (NS1, NS2, and M2-2). The structural proteins include 3 transmembrane surface proteins (G, F, SH), 2 matrix proteins (M and M2-1), and 3 nucleocapsid proteins (L, N, and P). G protein mediates the binding of the virus to the host cell, and F protein mediates the fusion of the virus with the host cell membrane, allowing the virus to enter the cell. Both G and F proteins are essential for viral replication and contain B cell and T cell epitopes, and they are the most important viral antigen proteins that stimulate humoral and cellular immunity in the body. The coding region of G protein varies greatly and can be divided into subtype A and subtype B based on its variation. The neutralizing antibodies induced by G protein are subtype specific. The coding region of F protein is highly conserved, and F proteins of subtype A and subtype B share at least 90% homology in terms of amino acid sequence; the neutralizing antibodies induced by F protein thus can inhibit RSV infection of both subtypes A and B at the same time. The structure of the F protein changes dynamically. It is first transcribed and translated into a single inactive polypeptide (F0) in the host cell, which is then cleaved for the first time by furin protease in the host cell, generating a partially cleaved prefusogenic protein. This is followed by a second furin cleavage, generating F2 and F1 subunits. The two subunits are linked into a monomer by two disulfide covalent bonds, and then the three monomers form a metastable functional prefusion F protein trimer. Thereafter, the trimer can undergo conformational rearrangement without further processing to form a thermodynamically stable postfusion F protein. The timing and cellular location of the two furin cleavages and conformational rearrangement are not yet fully understood, and the conditions that induce conformational rearrangement are also unclear. There are 6 antigenic epitopes related to neutralization activity on the surface of F protein, namely, I, II, III, IV, V, and Ø. Among them, epitopes I, II, III, and IV exist in F protein before and after fusion; V and Ø are antigenic sites specfic for F protein before fusion and thus are absent from F protein after fusion. Epidemiological studies have shown that RSV neutralizing antibodies can prevent severe RSV-ALRI; the monoclonal antibody against epitope Ø has a neutralizing activity which is 10 to 100 times that of monoclonal antibody against epitope II; and the monoclonal antibody against epitope VIII also exhibits a very high neutralizing activity. Therefore, most RSV neutralizing activities in serum are only directed against the antigenic sites of F protein before fusion. The inventors have recognized that, the development of RSV vaccines aims primarily at inducing neutralizing antibodies at a high titer, and the pre-fusion F protein with specific antigenic epitopes has become the hotspot of RSV vaccine targets. However, the pre-fusion F protein is essentially an unstable protein, and making multiple modifications thereto for stability without losing important antigenic epitopes would be one of the difficulties in RSV vaccine research and development.

### Summary of the invention

In some embodiments, the present disclosure provides an immunological composition comprising or encoding a human respiratory syncytial virus (hRSV) antigen, which is capable of inducing an effective neutralizing antibody response against human respiratory syncytial virus (hRSV).

In some aspects, the present disclosure provides an immunological composition comprising or encoding a human respiratory syncytial virus (hRSV) antigen, wherein the immunological composition is selected from a nucleic acid immunological composition, a polypeptide immunological composition, or a viral immunological composition.

In some embodiments, the antigen is a pre-fusion hRSV F protein, and the pre-fusion hRSV F protein has an amino acid sequence comprising a TM sequence and a multimerization element.

In some embodiments, the multimerization element has an amino acid sequence as set forth in SEQ ID NOs. 1-12 or SEQ ID NOs. 14-15.

In some embodiments, the multimerization element has an amino acid sequence as set forth in one of SEQ ID NO. 7 and SEQ ID NO. 12.

In some embodiments, the pre-fusion hRSV F protein variant has the P27 sequence replaced with a GS sequence.

In some embodiments, the GS sequence comprises (GnS)m, (GGGGS)o, GGSGGGGSGG, GGSGGGGG, GSGSGSGS, (Gly)p, (EAAAK)q, such as SEQ ID NOs. 48-61, or SEQ ID NO. 74 (wherein n is 1, 2, 3, 4, 5, 6, 7, 8, 9, 10, 15 or 20; m is 1, 2, 3, 4, 5, 6, 7, 8, 9, 10, 15 or 20; o is an integer from 1 to 5; p is an integer from 1 to 40; q is an integer from 1 to 5).

In some embodiments, the GS sequence has an amino acid sequence as set forth in SEQ ID NO. 74.

In some embodiments, the pre-fusion hRSV F protein variant has an amino acid sequence comprising at least one of an S46G substitution, an N67I substitution, an E92D substitution, a P102A substitution, an A149C substitution, an S155C substitution, an S190F substitution, a V207L substitution, an S290C substitution, an L373R substitution, an S215P substitution, an I379V substitution, an M447V substitution, a Y458C substitution, a K465Q substitution, a D486C substitution, and a D489C substitution.

In some embodiments, the pre-fusion hRSV F protein has an amino acid sequence comprising at least one of an S155C substitution, an S190F substitution, a V207L substitution, and an S290C substitution, and/or the pre-fusion hRSV F protein has the P27 sequence replaced with a GS sequence.

In some embodiments, the pre-fusion hRSV F protein has an amino acid sequence comprising an S155C substitution, an S190F substitution, a V207L substitution, and an S290C substitution; the pre-fusion hRSV F protein has the P27 sequence replaced with the GS sequence as set forth in SEQ ID NO. 74; the pre-fusion hRSV F protein comprises a multimerization element as set forth in one of SEQ ID NO. 7 and SEQ ID NO. 12.

In some embodiments, the pre-fusion hRSV F protein has an amino acid sequence comprising an S155C substitution, an S190F substitution, a V207L substitution, and an S290C substitution; the pre-fusion hRSV F protein has the P27 sequence replaced with the GS sequence as set forth in SEQ ID NO. 74; the pre-fusion hRSV F protein comprises a multimerization element as set forth in one of SEQ ID NO. 7 and SEQ ID NO. 12; the amino acid sequence of the pre-fusion hRSV F protein further comprises at least one of an S46G substitution, an N67I substitution, an E92D substitution, a P102A substitution, an A149C substitution, an L373R substitution, an S215P substitution, an I379V substitution, an M447V substitution, a Y458C substitution, a K465Q substitution, a D486C substitution, and a D489C substitution.

In some embodiments, the pre-fusion hRSV F protein has an amino acid sequence comprising at least one of an S155C substitution, an S190F substitution, a V207L substitution, an S290C substitution, a D486C substitution, and a D489C substitution.

In some embodiments, the pre-fusion hRSV F protein has an amino acid sequence comprising at least one of an S155C substitution, an S190F substitution, a V207L substitution, an S290C substitution, a D486C substitution, and a D489C substitution, and/or the pre-fusion hRSV F protein has the P27 sequence replaced with a GS sequence.

In some embodiments, the pre-fusion hRSV F protein has an amino acid sequence comprising an S155C substitution, an S190F substitution, a V207L substitution, an S290C substitution, a D486C substitution, and a D489C substitution, and/or the pre-fusion hRSV F protein has the P27 sequence replaced with a GS sequence as set forth in SEQ ID NO. 74, and/or the pre-fusion hRSV F protein comprises a multimerization element as set forth in one of SEQ ID NO. 7 and SEQ ID NO. 12.

In some embodiments, the pre-fusion hRSV F protein has an amino acid sequence comprising an S155C substitution, an S190F substitution, a V207L substitution, an S290C substitution, a D486C substitution, and a D489C substitution, and the pre-fusion hRSV F protein has the P27 sequence replaced with the GS sequence as set forth in SEQ ID NO. 74, and the pre-fusion hRSV F protein comprises a multimerization element as set forth in one of SEQ ID NO. 7 and SEQ ID NO. 12; the amino acid sequence of the pre-fusion hRSV F protein also comprises at least one of an S46G substitution, an N67I substitution, an E92D substitution, a P102A substitution, an A149C substitution, an L373R substitution, an S215P substitution, an I379V substitution, an M447V substitution, a Y458C substitution, and a K465Q substitution.

In some embodiments, the pre-fusion hRSV F protein variant comprises an amino acid sequence in which at least one of the amino acids in the F2 sequence, the RR1 sequence, the RR2 sequence, the 525-529 sequence or the 551-574 sequence is deleted sequentially or intermittently.

In some embodiments, the pre-fusion hRSV F protein has an amino acid sequence as set forth in SEQ ID NO. 62, SEQ ID NO. 64, SEQ ID NO. 66, SEQ ID NO. 68, SEQ ID NO. 70, SEQ ID NO. 72, SEQ ID NO. 76, or SEQ ID NO. 78.

In some embodiments, the pre-fusion hRSV F protein has an amino acid sequence as set forth in SEQ ID NO. 62, SEQ ID NO. 64, SEQ ID NO. 66, SEQ ID NO. 70, SEQ ID NO. 76, or SEQ ID NO. 78.

In some embodiments, the pre-fusion hRSV F protein has an amino acid sequence as set forth in SEQ ID NO. 62, SEQ ID NO. 66, SEQ ID NO. 70, SEQ ID NO. 76, or SEQ ID NO. 78.

In some embodiments, the multimerization element of the pre-fusion hRSV F protein can further be selected from the amino acid sequences as set forth in SEQ ID NOs. 1-12 and SEQ ID NOs. 14-15.

In some embodiments, the immunological composition is a nucleic acid immunological composition.

In some embodiments, the nucleic acid immunological composition comprises a nucleic acid molecule.

In some embodiments, the nucleic acid molecule comprises a DNA molecule and/or an RNA molecule.

In some embodiments, the DNA molecule comprises a stranded DNA molecule and/or a circular DNA molecule.

In some embodiments, the RNA molecule comprises an mRNA or a circular RNA.

In some embodiments, the mRNA molecule comprises an open reading frame (ORF), and the open reading frame (ORF) has a nucleotide sequence as set forth in SEQ ID NO. 63, SEQ ID NO. 65, SEQ ID NO. 67, SEQ ID NO. 69, SEQ ID NO. 71, SEQ ID NO. 73, SEQ ID NO. 77, SEQ ID NO. 79, or SEQ ID NOs. 84~103.

In some embodiments, the mRNA molecule comprises an open reading frame (ORF), and the open reading frame (ORF) has a nucleotide sequence as set forth in SEQ ID NO. 63, SEQ ID NO. 67, SEQ ID NO. 71, SEQ ID NO. 77, SEQ ID NO. 79, or SEQ ID NOs. 84~103.

In some embodiments, the mRNA molecule comprises an open reading frame (ORF), and the open reading frame (ORF) has a nucleotide sequence as set forth in SEQ ID NO. 63, SEQ ID NO. 71, SEQ ID NO. 77, SEQ ID NO. 79, SEQ ID NOs. 84-87, or SEQ ID NOs. 92-103.

In some embodiments, the mRNA molecule comprises an open reading frame (ORF), and the open reading frame (ORF) has a nucleotide sequence as set forth in SEQ ID NO. 63, SEQ ID NO. 77, SEQ ID NO. 79, SEQ ID NOs. 84-87, or SEQ ID NOs. 96-103.

In some embodiments, the mRNA molecule comprises an open reading frame (ORF), and the open reading frame (ORF) has a nucleotide sequence as set forth in SEQ ID NO. 67.

In some embodiments, the mRNA molecule comprises an open reading frame (ORF), and the open reading frame (ORF) has a nucleotide sequence as set forth in SEQ ID NO. 71.

In some embodiments, the multimerization element in the open reading frame (ORF) can also has a nucleotide sequence as set forth in SEQ ID NOs. 36-47, or SEQ ID NO. 13.

In some embodiments, the immunological composition comprises a delivery formulation; in some embodiments, the delivery formulation comprises lipid nanoparticles or cationic liposomes.

In some embodiments, the mRNA and a delivery formulation are mixed to form an immunological composition; the delivery formulation comprises lipid nanoparticles or cationic liposomes.

In some embodiments, the present disclosure provides an isolated mRNA, wherein the nucleic acid sequence of the coding region of the mRNA encodes a pre-fusion hRSV F protein, and the pre-fusion hRSV F protein has an amino acid sequence comprising a TM sequence and a multimerization element; preferably, the multimerization element has an amino acid sequence as set forth in SEQ ID NOs. 1-12, or SEQ ID NOs. 14-15; preferably, the multimerization element has an amino acid sequence as set forth in one of SEQ ID NO. 7 and SEQ ID NO. 12.

In some embodiments, the mRNA further comprises at least one of a 5' cap, a 5' UTR, a 3' UTR, and a 3' polyA tail.

In some embodiments, the 5' cap is selected from ARCA, mCAP, dmCAP, tmCAP, m7G(5"")ppp(5"")(2""OMeA)pG, m7(3 ""OMeG)(5"")ppp(5" ")(2" "OMeA)pG, m7(3""OMeG)(5"")ppp(5"")(2""OMeG)pG, dmCAP or m7G(5" ")ppp(5" ")(2" "OMeG)pG.

In some embodiments, the 5' cap is m7Gppp(5' )(2' - OMeA)pG.

In some embodiments, the 3' polyA tail has a length of 50-200.

In some embodiments, the 3' polyA tail has a length of 80-200.

In some embodiments, the 3' polyA tail has a length of 80 to 120. In some embodiments, the 3' polyA tail has a length of 120.

In some embodiments, the 5'UTR preferably has a length of 10 to 200 nucleotides.

In some embodiments, the 5'UTR has a length of 15 to 100 nucleotides.

In some embodiments, the 5'UTR has a nucleotide sequence as set forth in SEQ ID NO. 16-18.

In some embodiments, the 5'UTR has a nucleotide sequence as set forth in SEQ ID NO. 16.

In some embodiments, the 3'UTR has a sequence as set forth in SEQ ID NO. 19-21.

In some embodiments, the 3'UTR has a sequence as set forth in SEQ ID NO. 19.

In some embodiments, one or more uridines in the mRNA are replaced with modified nucleosides.

In some embodiments, the modified nucleoside is pseudouridine (ψ), N1-methyl-pseudouridine (m1ψ), or 5-methyl-uridine (m5U).

In some embodiments, the modified nucleoside is N1-methyl-pseudouridine (m1Ψ).

In some embodiments, the present disclosure provides an isolated DNA, wherein the DNA is obtained by reverse transcription of the isolated mRNA, or the DNA is a sequence that can be transcribed into the isolated mRNA.

In some embodiments, the present disclosure provides a biological material, comprising any one of an expression cassette, a vector, an engineered bacterium or a cell line, wherein the biological material contains or expresses the isolated mRNA or the isolated DNA.

### BRIEF DESCRIPTION OF THE DRAWINGS

In order to more clearly illustrate the specific embodiments of the present disclosure or the technical solutions in the prior art, the drawings that need to be used in the description of the specific embodiments or the prior art will be briefly introduced below. Apparently, the drawings in the following description illustrate some embodiments of the present disclosure. For those of ordinary skill in the art, other drawings can be obtained based on these drawings without exerting inventive efforts.
FIG. 1 shows a schematic diagram of the wild-type hRSV F protein.
FIG. 2 shows a schematic diagram of an hRSV F protein variant.
FIG. 3 shows a schematic diagram of the evaluation process of the experiment in mouse.
FIG. 4 shows the detection of TNFα+ CD4+ and TNF-α+ CD8+ T cells by flow cytometry in Example 5.
FIG. 5 shows the determination of IFN-γ and L4 in the cell culture supernatant by ELISA in Example 5.
FIG. 6 shows the RSV neutralizing antibody titer in mouse serum in Example 5.
FIG. 7 shows a statistical graph regarding the titer of RSV-F protein-specific IgG antibody in mouse serum determined by ELISA in Example 6.
FIG. 8 shows the detection of TNFα+ CD4+ and TNF-α+ CD8+ T cells by flow cytometry in Example 6.
FIG. 9 shows the determination of IFN-γ and IL2 in the cell culture supernatant by ELISA in Example 6.
FIG. 10 shows the determination of IL4 and IL10 in the cell culture supernatant by ELISA in Example 6.
FIG. 11 shows the RSV neutralizing antibody titer in mouse serum in Example 6.
FIG. 12 shows the detection of TNFα+ CD4+ and TNF-α+ CD8+ T cells by flow cytometry in Example 7.
FIG. 13 shows the detection of IFN-γ+ CD4+ and IFN-γ+ CD8+ T cells by flow cytometry in Example 7.
FIG. 14 shows the determination of IFN-γ and IL2 in the cell culture supernatant by ELISA in Example 7.
FIG. 15 shows the determination of IL4 and IL10 in the cell culture supernatant by flow cytometry in Example 7.
FIG. 16 shows the RSV neutralizing antibody titer in mouse serum in Example 7.
FIG. 17 shows the detection of TNFα+ CD4+ and TNF-α+ CD8+ T cells by flow cytometry in Example 8.
FIG. 18 shows the detection of IFN-γ+ CD4+ and IFN-γ+ CD8+ T cells by flow cytometry in Example 8.
FIG. 19 shows the determination of IFN-γ and IL2 in the cell culture supernatant by ELISA in Example 8.
FIG. 20 shows the determination of IL4 and IL10 in the cell culture supernatant by ELISA in Example 8.
FIG. 21 shows the RSV neutralizing antibody titer in mouse serum in Example 8.
FIG. 22 shows the RSV neutralizing antibody titer in mouse serum in Example 9.
FIG. 23 shows the RSV neutralizing antibody titer in mouse serum in Example 11.
FIG. 24 shows the RSV neutralizing antibody titer in mouse serum in Example 12.
FIG. 25 shows the RSV neutralizing antibody titer in mouse serum in Example 12.
FIG. 26 shows the RSV neutralizing antibody titer in mouse serum in Example 12.
FIG. 27 is a graph showing the trend of change in mouse weight in Example 13.
FIG. 28 is a graph showing the trend of change rate in mouse weight in Example 13.
FIG. 29 shows a statistical graph regarding the titer of RSV-F protein-specific IgG antibody in mouse serum determined by ELISA in Example 13.
FIG. 30 shows the results of titer determination of neutralizing antibody against RSV-A/B strain in mouse serum in Example 13.
FIG. 31 shows the results of virus titer determination in the lung tissue of mouse infected with RSV in Example 13.
FIG. 32 shows the results of lung tissue pathology of mouse infected with RSV in Example 13.

### DETAILED DESCRIPTION

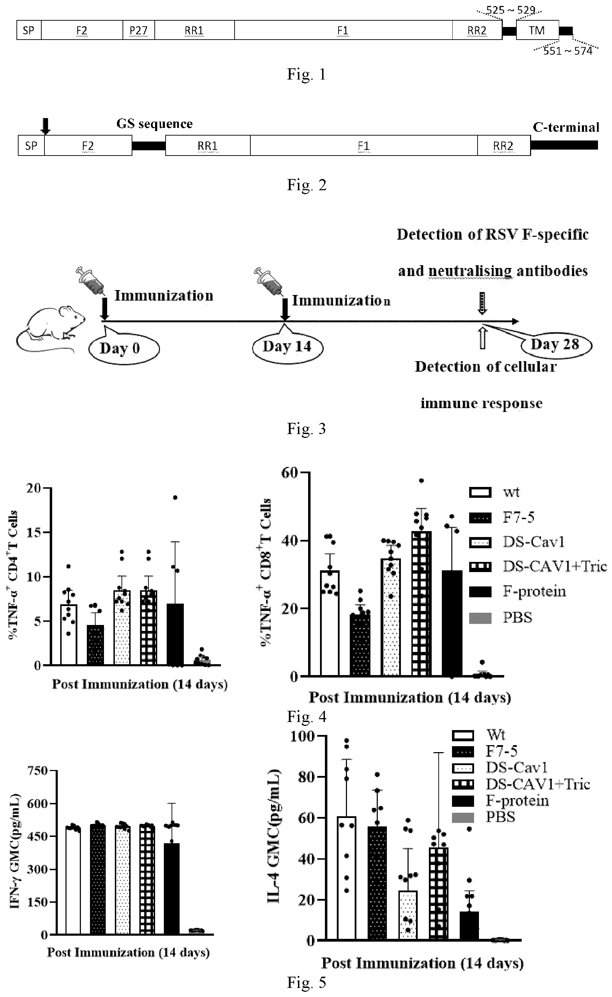

FIG. 1 shows a schematic diagram of the wild-type hRSV F protein before fusion. FIG. 2 shows a schematic diagram of one of the pre-fusion hRSV F proteins (pre-fusion hRSV F protein variant) of the antigen of the immunological composition according to the present disclosure.

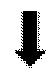 represents furin protease cleavage; SP represents the signal peptide sequence; P27 represents the peptide sequence generated after cleavage and removal of 27aa; RR1 represents repeat folding region 1, spanning 137 to 216 amino acid residues of the wild type pre-fusion hRSV F protein, including the fusion peptide and the heptad repeat sequence A (HRA); RR2 represents repeat folding region 2, which forms the C-terminal stem in the prefusion hRSV F protein spike, including the heptad repeat sequence B (HRB) relocated to the other side of the prefusion RSV F protein head; the 525-529 sequence represents a stretch of sequence connecting RR2 and TM; TM represents the transmembrane region sequence; the 551-574 sequence represents a stretch of sequence conjugated to the C-terminus of the TM sequence.

F2 generally contains amino acid residues 26 to 109 of the F0 precursor; F1 generally contains amino acid residues 137 to 574 of the F0 precursor; F1 and F2 are linked by disulfide bonds to form a heterodimer, which is called the RSV F "protomer".

"pre-fusion hRSV F protein" or "pre-fusion hRSV F protein (wild type)" according to the present disclosure refers to the wild type pre-fusion hRSV F protein without artificial modification or variation.

The C-terminus shown in FIG. 2 may be missing or selected from at least one of a TM sequence, a 525-529 sequence, a 551-574 sequence, and a multimerization element. In some embodiments, the C-terminus of the pre-fusion hRSV F protein variant according to the present disclosure is missing. In some embodiments, the C-terminus of the pre-fusion hRSV F protein variant is a TM sequence. In some embodiments, the C-terminus of the pre-fusion hRSV F protein variant is a TM sequence and a multimerization element connected in sequence, wherein the C-terminus of the TM sequence is connected to the N-terminus of the multimerization element. In some embodiments, the C-terminus of the pre-fusion hRSV F protein variant is a TM sequence. In some embodiments, the C-terminus of the pre-fusion hRSV F protein variant is a TM sequence, a 551-574 sequence, and a multimerization element connected in sequence, wherein the C-terminus of the TM sequence is connected to the 551-574 sequence, and the C-terminus of the 551-574 sequence is connected to the N-terminus of the multimerization element. In some embodiments, the C-terminus of the pre-fusion hRSV F protein variant is a multimerization element.

The multimerization elements according to the present disclosure include dimerization elements, trimerization elements, tetramerization elements and oligomerization elements, which, when used in combination with the hRSV F protein according to the present disclosure, can lead to the formation of a multimeric hRSV F protein complex. The multimerization element can be located at the N-terminus or C-terminus of the hRSV F protein, and its coding sequence is usually placed in the frame 5' or 3' of the coding sequence at the nucleic acid level.

In some embodiments, the dimerization element can be selected from, for example, a dimerization element/domain of a heat shock protein, an immunoglobulin Fc domain, and a leucine zipper (a dimerization domain of a basic region leucine zipper-like transcription factor), and the specific amino acid sequence is set forth in, e.g., SEQ ID NO. 1-2. The trimerization and tetramerization elements can be selected from, for example, an engineered leucine zipper (an engineered helical coiled peptide in a parallel trimer state), a fibritin foldon domain of Enterobacteria phage T4, GCN4PLL, CCN4-PLI, p53, GCN4, and the specific amino acid sequence is set forth in, e.g., SEQ ID NOs. 3-12 (trimerization) and SEQ ID NOs. 14-15 (tetramerization)).

The "pre-fusion hRSV F protein vairant" according to the present disclosure refers to an F protein with artificial mutatation and modification, including but not limited to amino acid residue substitution, amino acid residue insertion and/or addition, amino acid residue deletion and covalent modification, etc., which is obtained by mutation and engineerring of the wild-type pre-fusion hRSV F protein to conform with the amino acid sequence of the pre-fusion hRSV F protein. In some embodiments of the present disclosure, if "a pre-fusion hRSV F protein" is not described as "a pre-fusion hRSV F protein wild type" or "a pre-fusion hRSV F protein (wild type)", then "a pre-fusion hRSV F protein" is a pre-fusion hRSV F protein variant.

In some embodiments, the GS sequence comprises, for example, (GnS)m, (GGGGS), GGSGGGGSGG, GGSGGGGG, GSGSGSGS, (Gly)p, (EAAAK)q, SEQ ID NOs. 48-61, or SEQ ID NO. 74 (wherein n is 1, 2, 3, 4, 5, 6, 7, 8, 9, 10, 15 or 20; m is 1, 2, 3, 4, 5, 6, 7, 8, 9, 10, 15 or 20; o is an integer from 1 to 5; p is an integer from 1 to 40; q is an integer from 1 to 5). Further, in some embodiments, the GS sequence has an amino acid sequence as set forth in SEQ ID NO. 74, or, the nucleotide sequence encoding the amino acid sequence as set forth in SEQ ID NO. 74 is optimized.

In some embodiments, the present disclosure provides an immunological composition comprising or encoding a human respiratory syncytial virus (hRSV) antigen, which is capable of inducing an effective neutralizing antibody response against human respiratory syncytial virus (hRSV).

The present disclosure provides an immunological composition comprising or encoding a human respiratory syncytial virus (hRSV) antigen, wherein the immunological composition is selected from a nucleic acid immunological composition, a polypeptide immunological composition, or a viral immunological composition.

In some embodiments, the antigen is a pre-fusion hRSV F protein, and the pre-fusion hRSV F protein has an amino acid sequence comprising a TM sequence and a multimerization element.

In some embodiments, the multimerization element has an amino acid sequence as set forth in SEQ ID NOs. 1-12 or SEQ ID NOs. 14-15.

In some embodiments, the multimerization element has an amino acid sequence as set forth in one of SEQ ID NO. 7 and SEQ ID NO. 12.

In some embodiments, the pre-fusion hRSV F protein variant has the P27 sequence replaced with a GS sequence.

In some embodiments, the GS sequence comprises (GnS)m, (GGGGS)o, GGSGGGGSGG, GGSGGGGG, GSGSGSGS, (Gly)p, (EAAAK)q, SEQ ID NOs. 48-61, or SEQ ID NO. 74 (wherein n is 1, 2, 3, 4, 5, 6, 7, 8, 9, 10, 15 or 20; m is 1, 2, 3, 4, 5, 6, 7, 8, 9, 10, 15 or 20; o is an integer from 1 to 5; p is an integer from 1 to 40; q is an integer from 1 to 5).

In some embodiments, the GS sequence has an amino acid sequence as set forth in SEQ ID NO. 74.

In some embodiments, the pre-fusion hRSV F protein variant has an amino acid sequence comprising at least one of an S46G substitution, an N67I substitution, an E92D substitution, a P102A substitution, an A149C substitution, an S155C substitution, an S190F substitution, a V207L substitution, an S290C substitution, an L373R substitution, an S215P substitution, an I379V substitution, an M447V substitution, a Y458C substitution, a K465Q substitution, a D486C substitution, and a D489C substitution.

In some embodiments, the pre-fusion hRSV F protein has an amino acid sequence comprising at least one of an S155C substitution, an S190F substitution, a V207L substitution, an S290C substitution, and/or the pre-fusion hRSV F protein has the P27 sequence replaced with a GS sequence.

In some embodiments, the pre-fusion hRSV F protein has an amino acid sequence comprising an S155C substitution, an S190F substitution, a V207L substitution, and an S290C substitution; the pre-fusion hRSV F protein has the P27 sequence replaced with the GS sequence as set forth in SEQ ID NO. 74; the pre-fusion hRSV F protein comprises a multimerization element as set forth in one of SEQ ID NO. 7 and SEQ ID NO. 12.

In some embodiments, the pre-fusion hRSV F protein has an amino acid sequence comprising an S155C substitution, an S190F substitution, a V207L substitution, and an S290C substitution; the pre-fusion hRSV F protein has the P27 sequence replaced with the GS sequence as set forth in SEQ ID NO. 74; the pre-fusion hRSV F protein comprises a multimerization element as set forth in one of SEQ ID NO. 7 and SEQ ID NO. 12; the amino acid sequence of the pre-fusion hRSV F protein also comprises at least one of an S46G substitution, an N67I substitution, an E92D substitution, a P102A substitution, an A149C substitution, an L373R substitution, an S215P substitution, an I379V substitution, an M447V substitution, a Y458C substitution, a K465Q substitution, a D486C substitution, and a D489C substitution.

In some embodiments, the pre-fusion hRSV F protein has an amino acid sequence comprising an S155C substitution, an S190F substitution, a V207L substitution, and an S290C substitution; the pre-fusion hRSV F protein has the P27 sequence replaced with the GS sequence as set forth in SEQ ID NO. 74; the pre-fusion hRSV F protein comprises a multimerization element as set forth in one of SEQ ID NO. 7 and SEQ ID NO. 12; the pre-fusion hRSV F protein variant comprises an amino acid sequence in which at least one of the amino acids in the F2 sequence, the RR1 sequence, the RR2 sequence, the 525-529 sequence or the 551-574 sequence is deleted sequentially or intermittently.

In some embodiments, the pre-fusion hRSV F protein has an amino acid sequence comprising at least one of an S155C substitution, an S190F substitution, a V207L substitution, an S290C substitution, a D486C substitution, and a D489C substitution, and/or the pre-fusion hRSV F protein has the P27 sequence replaced with a GS sequence.

In some embodiments, the pre-fusion hRSV F protein has an amino acid sequence comprising an S155C substitution, an S190F substitution, a V207L substitution, an S290C substitution, a D486C substitution, and a D489C substitution, and the pre-fusion hRSV F protein has the P27 sequence replaced with the GS sequence as set forth in SEQ ID NO. 74, and the pre-fusion hRSV F protein comprises a multimerization element as set forth in one of SEQ ID NO. 7 and SEQ ID NO. 12.

In some embodiments, the pre-fusion hRSV F protein has an amino acid sequence comprising an S155C substitution, an S190F substitution, a V207L substitution, an S290C substitution, a D486C substitution, and a D489C substitution;
the pre-fusion hRSV F protein has the P27 sequence replaced with the GS sequence as set forth in SEQ ID NO. 74;
the pre-fusion hRSV F protein comprises a multimerization element as set forth in one of SEQ ID NO. 7 and SEQ ID NO. 12;
the amino acid sequence of the pre-fusion hRSV F protein also comprises at least one of an S46G substitution, an N67I substitution, an E92D substitution, a P102A substitution, an A149C substitution, an L373R substitution, an S215P substitution, an I379V substitution, an M447V substitution, a Y458C substitution, and a K465Q substitution.

In some embodiments, the pre-fusion hRSV F protein has an amino acid sequence comprising an S155C substitution, an S190F substitution, a V207L substitution, an S290C substitution, a D486C substitution, and a D489C substitution;
the pre-fusion hRSV F protein has the P27 sequence replaced with the GS sequence as set forth in SEQ ID NO. 74;
the pre-fusion hRSV F protein comprises a multimerization element as set forth in one of SEQ ID NO. 7 and SEQ ID NO. 12;
the pre-fusion hRSV F protein variant comprises an amino acid sequence in which at least one of the amino acids in the F2 sequence, the RR1 sequence, the RR2 sequence, the 525-529 sequence or the 551-574 sequence is deleted sequentially or intermittently.

In some embodiments, the pre-fusion hRSV F protein variant comprises an amino acid sequence in which at least one of the amino acids in the F2 sequence, the RR1 sequence, the RR2 sequence, the 525-529 sequence or the 551-574 sequence is deleted sequentially or intermittently.

In some embodiments, the pre-fusion hRSV F protein variant has an F2 sequence in which at least 1, 2, 3, 4, 5, 6, 7, 8, 9, 10, 11, or 12 amino acids between positions 98 to 109 are deleted sequentially or intermittently.

In some embodiments, the pre-fusion hRSV F protein variant has an RR1 sequence in which at least 1, 2, 3, 4, 5, 6, 7, or 8 amino acids between positions 137 to 144 are deleted sequentially or intermittently.

In some embodiments, the pre-fusion hRSV F protein variant has an RR2 sequence in which at least 1, 2, 3, 4, 5, 6, 7, 8, 9, 10, 11, 12, 13, 14, 15, 16, 17, 18, 19, 20, or 21 amino acids between positions 504 to 524 are deleted sequentially or intermittently.

In some embodiments, the pre-fusion hRSV F protein variant has a 525-529 sequence in which at least 1, 2, 3, 4, or 5 amino acids are deleted sequentially or intermittently.

In some embodiments, the pre-fusion hRSV F protein variant has a 551-574 sequence in which at least 1, 2, 3, 4, 5, 6, 7, 8, 9, 10, 11, 12, 13, or 14 amino acids are deleted sequentially or intermittently.

The positioning of the amino acid sequence according to the present disclosure, such as S46G substitution, E92D substitution, P102A substitution, A149C substitution, L373R substitution, I379V substitution, M447V substitution, Y458C substitution, K465Q substitution, D486C substitution, and D489C substitution, makes reference to the full-length amino acid sequence of pre-fusion hRSV F protein wild type as shown in Fig. 1. For example, the S46G substitution refers to replacing the S amino acid residue of the 46^{th} position of the full-length amino acid sequence of pre-fusion hRSV F protein wild type as shown in Fig. 1 with G amino acid residue, on the basis of pre-fusion hRSV F protein wild type, and this mutation occurs in F2 sequence. For example, the 525-529 sequence refers to linking RR2 sequence and TM sequence on the pre-fusion hRSV F protein wild type as shown in Fig. 1, on the basis of pre-fusion hRSV F protein wild type. For example, the 551-574 sequence refers to linking to the C-terminus of the TM sequence on the pre-fusion hRSV F protein wild type as shown in Fig. 1, on the basis of pre-fusion hRSV F protein wild type.

In some embodiments, the pre-fusion hRSV F protein has an amino acid sequence as set forth in SEQ ID NO. 62, SEQ ID NO. 76 or SEQ ID NO. 78, or comprises an amino acid sequence that is at least 80% identical to SEQ ID NO. 62, SEQ ID NO. 76 or SEQ ID NO. 78, for example, it can be but is not limited to an amino acid sequence that is at least 80%, 85%, 90%, 95% or 98% identical to SEQ ID NO. 62, SEQ ID NO. 76 or SEQ ID NO. 78.

In some embodiments, the hRSV F protein variant in the stabilized pre-fusion form has an amino acid sequence as set forth in SEQ ID NO. 66, or comprises an amino acid sequence that is at least 80% identical to SEQ ID NO. 66, for example, it can be but is not limited to an amino acid sequence that is at least 80%, 85%, 90%, 95% or 98% identical to SEQ ID NO. 66.

In some embodiments, the hRSV F protein variant in the stabilized pre-fusion form has an amino acid sequence as set forth in SEQ ID NO. 70, or comprises an amino acid sequence that is at least 80% identical to SEQ ID NO. 70, for example, it can be but is not limited to an amino acid sequence that is at least 80%, 85%, 90%, 95% or 98% identical to SEQ ID NO. 70.

In some embodiments, the hRSV F protein variant in the stabilized pre-fusion form has an amino acid sequence as set forth in SEQ ID NO. 64, or comprises an amino acid sequence that is at least 80% identical to SEQ ID NO. 64, for example, it can be but is not limited to an amino acid sequence that is at least 80%, 85%, 90%, 95% or 98% identical to SEQ ID NO. 64.

In some embodiments, the hRSV F protein variant in the stabilized pre-fusion form has an amino acid sequence as set forth in SEQ ID NO. 68, or comprises an amino acid sequence that is at least 80% identical to SEQ ID NO. 68, for example, it can be but is not limited to an amino acid sequence that is at least 80%, 85%, 90%, 95% or 98% identical to SEQ ID NO. 68.

In some embodiments, the hRSV F protein variant in the stabilized pre-fusion form has an amino acid sequence as set forth in SEQ ID NO. 72, or comprises an amino acid sequence that is at least 80% identical to SEQ ID NO. 72, for example, it can be but is not limited to an amino acid sequence that is at least 80%, 85%, 90%, 95% or 98% identical to SEQ ID NO. 72.

In some embodiments, the pre-fusion hRSV F protein variant comprises an amino acid sequence in which at least one of the amino acids in the F2 sequence, the RR1 sequence, the RR2 sequence, the 525-529 sequence or the 551-574 sequence is deleted sequentially or intermittently.

In some embodiments, the pre-fusion hRSV F protein variant has an F2 sequence in which at least 1, 2, 3, 4, 5, 6, 7, 8, 9, 10, 11, or 12 amino acids between positions 98 to 109 are deleted sequentially or intermittently.

In some embodiments, the pre-fusion hRSV F protein variant has an RR1 sequence in which at least 1, 2, 3, 4, 5, 6, 7, or 8 amino acids between positions 137 to 144 are deleted sequentially or intermittently.

In some embodiments, the pre-fusion hRSV F protein variant has an RR2 sequence in which at least 1, 2, 3, 4, 5, 6, 7, 8, 9, 10, 11, 12, 13, 14, 15, 16, 17, 18, 19, 20, or 21 amino acids between positions 504 to 524 are deleted sequentially or intermittently.

In some embodiments, the pre-fusion hRSV F protein variant has a 525-529 sequence in which at least 1, 2, 3, 4, or 5 amino acids are deleted sequentially or intermittently.

In some embodiments, the pre-fusion hRSV F protein variant has a 551-574 sequence in which at least 1, 2, 3, 4, 5, 6, 7, 8, 9, 10, 11, 12, 13, or 14 amino acids are deleted sequentially or intermittently.

In some embodiments, the pre-fusion hRSV F protein has an amino acid sequence as set forth in SEQ ID NO. 62, SEQ ID NO. 64, SEQ ID NO. 66, SEQ ID NO. 68, SEQ ID NO. 70, SEQ ID NO. 72, SEQ ID NO. 76, or SEQ ID NO. 78; and/or
the pre-fusion hRSV F protein has an F2 sequence in which at least 1, 2, 3, 4, 5, 6, 7, 8, 9, 10, 11, or 12 amino acids between positions 98 to 109 are deleted sequentially or intermittently; and/or
the pre-fusion hRSV F protein has an RR1 sequence in which at least 1, 2, 3, 4, 5, 6, 7, or 8 amino acids between positions 137 to 144 are deleted sequentially or intermittently; and/or
the pre-fusion hRSV F protein has an RR2 sequence in which at least 1, 2, 3, 4, 5, 6, 7, 8, 9, 10, 11, 12, 13, 14, 15, 16, 17, 18, 19, 20, or 21 amino acids between positions 504 to 524 are deleted sequentially or intermittently; and/or
the pre-fusion hRSV F protein has a 525-529 sequence in which at least 1, 2, 3, 4, or 5 amino acids are deleted sequentially or intermittently; and/or
the pre-fusion hRSV F protein has a 551-574 sequence in which at least 1, 2, 3, 4, 5, 6, 7, 8, 9, 10, 11, 12, 13, or 14 amino acids are deleted sequentially or intermittently.

In some embodiments, the pre-fusion hRSV F protein has an amino acid sequence as set forth in SEQ ID NO. 62, SEQ ID NO. 64, SEQ ID NO. 66, SEQ ID NO. 70, SEQ ID NO. 76, or SEQ ID NO. 78; and/or
the pre-fusion hRSV F protein has an F2 sequence in which at least 1, 2, 3, 4, 5, 6, 7, 8, 9, 10, 11, or 12 amino acids between positions 98 to 109 are deleted sequentially or intermittently; and/or
the pre-fusion hRSV F protein has an RR1 sequence in which at least 1, 2, 3, 4, 5, 6, 7, or 8 amino acids between positions 137 to 144 are deleted sequentially or intermittently; and/or
the pre-fusion hRSV F protein has an RR2 sequence in which at least 1, 2, 3, 4, 5, 6, 7, 8, 9, 10, 11, 12, 13, 14, 15, 16, 17, 18, 19, 20, or 21 amino acids between positions 504 to 524 are deleted sequentially or intermittently; and/or
the pre-fusion hRSV F protein has a 525-529 sequence in which at least 1, 2, 3, 4, or 5 amino acids are deleted sequentially or intermittently; and/or
the pre-fusion hRSV F protein has a 551-574 sequence in which at least 1, 2, 3, 4, 5, 6, 7, 8, 9, 10, 11, 12, 13, or 14 amino acids are deleted sequentially or intermittently.

In some embodiments, the pre-fusion hRSV F protein has an amino acid sequence as set forth in SEQ ID NO. 62, SEQ ID NO. 66, SEQ ID NO. 70, SEQ ID NO. 76, or SEQ ID NO. 78; and/or
the pre-fusion hRSV F protein has an F2 sequence in which at least 1, 2, 3, 4, 5, 6, 7, 8, 9, 10, 11, or 12 amino acids between positions 98 to 109 are deleted sequentially or intermittently; and/or
the pre-fusion hRSV F protein has an RR1 sequence in which at least 1, 2, 3, 4, 5, 6, 7, or 8 amino acids between positions 137 to 144 are deleted sequentially or intermittently; and/or
the pre-fusion hRSV F protein has an RR2 sequence in which at least 1, 2, 3, 4, 5, 6, 7, 8, 9, 10, 11, 12, 13, 14, 15, 16, 17, 18, 19, 20, or 21 amino acids between positions 504 to 524 are deleted sequentially or intermittently; and/or
the pre-fusion hRSV F protein has a 525-529 sequence in which at least 1, 2, 3, 4, or 5 amino acids are deleted sequentially or intermittently; and/or
the pre-fusion hRSV F protein has a 551-574 sequence in which at least 1, 2, 3, 4, 5, 6, 7, 8, 9, 10, 11, 12, 13, or 14 amino acids are deleted sequentially or intermittently.

In some embodiments, the multimerization element of the pre-fusion hRSV F protein can also be selected from the amino acid sequences as set forth in SEQ ID NOs. 1-12 and SEQ ID NOs. 14-15.

In some embodiments, the immunological composition encoding human respiratory syncytial virus (hRSV) antigen is a nucleic acid immunological composition; the nucleic acid immunological composition comprises a nucleic acid molecule; the nucleic acid molecule comprises a DNA molecule and/or an RNA molecule.

In some embodiments, the DNA molecule comprises a stranded DNA molecule and/or a circular DNA molecule.

In some embodiments, the RNA molecule comprises an mRNA or a circular RNA.

In some embodiments, the mRNA molecule comprises an open reading frame (ORF), and the open reading frame (ORF) has a nucleotide sequence as set forth in SEQ ID NO. 63, SEQ ID NO. 65, SEQ ID NO. 67, SEQ ID NO. 69, SEQ ID NO. 71, SEQ ID NO. 73, SEQ ID NO. 77, SEQ ID NO. 79, or SEQ ID NOs. 84-103, or, comprises a nucleotide sequence that is at least 80% identical to SEQ ID NO. 63, SEQ ID NO. 65, SEQ ID NO. 67, SEQ ID NO. 69, SEQ ID NO. 71, SEQ ID NO. 73, SEQ ID NO. 77, SEQ ID NO. 79, or SEQ ID NOs. 84-103. For example, the open reading frame (ORF) can, but is not limited to, comprise a nucleotide sequence that is at least 80%, 85%, 90%, 95% or 98% identical to SEQ ID NO. 63, SEQ ID NO. 65, SEQ ID NO. 67, SEQ ID NO. 69, SEQ ID NO. 71, SEQ ID NO. 73, SEQ ID NO. 77, SEQ ID NO. 79, or SEQ ID NOs. 84-103.

In some embodiments, the mRNA molecule comprises an open reading frame (ORF), and the open reading frame (ORF) has a nucleotide sequence as set forth in SEQ ID NO. 63, SEQ ID NO. 67, SEQ ID NO. 71, SEQ ID NO. 77, SEQ ID NO. 79, or SEQ ID NOs. 84-103, or, comprises a nucleotide sequence that is at least 80% identical to SEQ ID NO. 63, SEQ ID NO. 67, SEQ ID NO. 71, SEQ ID NO. 77, SEQ ID NO. 79, or SEQ ID NOs. 84-103. For example, the open reading frame (ORF) can, but is not limited to, comprise a nucleotide sequence that is at least 80%, 85%, 90%, 95% or 98% identical to SEQ ID NO. 63, SEQ ID NO. 67, SEQ ID NO. 71, SEQ ID NO. 77, SEQ ID NO. 79, or SEQ ID NOs. 84-103.

In some embodiments, the mRNA molecule comprises an open reading frame (ORF), and the open reading frame (ORF) has a nucleotide sequence as set forth in SEQ ID NO. 63, SEQ ID NO. 71, SEQ ID NO. 77, SEQ ID NO. 79, SEQ ID NOs. 84-87, or SEQ ID NOs. 92-103, or, comprises a nucleotide sequence that is at least 80% identical to SEQ ID NO. 63, SEQ ID NO. 71, SEQ ID NO. 77, SEQ ID NO. 79, SEQ ID NOs. 84-87, or SEQ ID NOs. 92-103. For example, the open reading frame (ORF) can, but is not limited to, comprise a nucleotide sequence that is at least 80%, 85%, 90%, 95% or 98% identical to SEQ ID NO. 63, SEQ ID NO. 71, SEQ ID NO. 77, SEQ ID NO. 79, SEQ ID NOs. 84-87, or SEQ ID NOs. 92-103.

In some embodiments, the mRNA molecule comprises an open reading frame (ORF), and the open reading frame (ORF) has a nucleotide sequence as set forth in SEQ ID NO. 63, SEQ ID NO. 77, SEQ ID NO. 79, SEQ ID NOs. 84-87, or SEQ ID NOs. 96-103, or, comprises a nucleotide sequence that is at least 80% identical to SEQ ID NO. 63, SEQ ID NO. 77, SEQ ID NO. 79, SEQ ID NOs. 84-87, or SEQ ID NOs. 96-103. For example, the open reading frame (ORF) can, but is not limited to, comprise a nucleotide sequence that is at least 80%, 85%, 90%, 95% or 98% identical to SEQ ID NO. 63, SEQ ID NO. 77, SEQ ID NO. 79, SEQ ID NOs. 84-87, or SEQ ID NOs. 96-103.

In some embodiments, the mRNA molecule comprises an open reading frame (ORF), and the open reading frame (ORF) has a nucleotide sequence as set forth in SEQ ID NO. 63, or SEQ ID NOs. 84-87, or, comprises a nucleotide sequence that is at least 80% identical to SEQ ID NO. 63, or SEQ ID NOs. 84-87. For example, the open reading frame (ORF) can, but is not limited to, comprise a nucleotide sequence that is at least 80%, 85%, 90%, 95% or 98% identical to SEQ ID NO. 63, or SEQ ID NOs. 84-87.

In some embodiments, the mRNA molecule comprises an open reading frame (ORF), and the open reading frame (ORF) has a nucleotide sequence as set forth in SEQ ID NO. 63, or, comprises a nucleotide sequence that is at least 80% identical to SEQ ID NO. 63. For example, the open reading frame (ORF) can, but is not limited to, comprise a nucleotide sequence that is at least 80%, 85%, 90%, 95% or 98% identical to SEQ ID NO. 63.

In some embodiments, the mRNA molecule comprises an open reading frame (ORF), and the open reading frame (ORF) has a nucleotide sequence as set forth in SEQ ID NO. 77, or SEQ ID NOs. 96-99, or, comprises a nucleotide sequence that is at least 80% identical to SEQ ID NO. 77, or SEQ ID NOs. 96-99. For example, the open reading frame (ORF) can, but is not limited to, comprise a nucleotide sequence that is at least 80%, 85%, 90%, 95% or 98% identical to SEQ ID NO. 77, or SEQ ID NOs. 96-99.

In some embodiments, the mRNA molecule comprises an open reading frame (ORF), and the open reading frame (ORF) has a nucleotide sequence as set forth in SEQ ID NO. 77, or, comprises a nucleotide sequence that is at least 80% identical to SEQ ID NO. 77. For example, the open reading frame (ORF) can, but is not limited to, comprise a nucleotide sequence that is at least 80%, 85%, 90%, 95% or 98% identical to SEQ ID NO. 77.

In some embodiments, the mRNA molecule comprises an open reading frame (ORF), and the open reading frame (ORF) has a nucleotide sequence as set forth in SEQ ID NO. 79, or SEQ ID NOs. 100-103, or, comprises a nucleotide sequence that is at least 80% identical to SEQ ID NO. 79, or SEQ ID NOs. 100-103. For example, the open reading frame (ORF) can, but is not limited to, comprise a nucleotide sequence that is at least 80%, 85%, 90%, 95% or 98% identical to SEQ ID NO. 79, or SEQ ID NOs. 100-103.

In some embodiments, the mRNA molecule comprises an open reading frame (ORF), and the open reading frame (ORF) has a nucleotide sequence as set forth in SEQ ID NO. 79, or, comprises a nucleotide sequence that is at least 80% identical to SEQ ID NO. 79. For example, the open reading frame (ORF) can, but is not limited to, comprise a nucleotide sequence that is at least 80%, 85%, 90%, 95% or 98% identical to SEQ ID NO. 79.

In some embodiments, the mRNA molecule comprises an open reading frame (ORF), and the open reading frame (ORF) has a nucleotide sequence as set forth in SEQ ID NO. 65, or, comprises a nucleotide sequence that is at least 80% identical to SEQ ID NO. 65. For example, the open reading frame (ORF) can, but is not limited to, comprise a nucleotide sequence that is at least 80%, 85%, 90%, 95% or 98% identical to SEQ ID NO. 65.

In some embodiments, the mRNA molecule comprises an open reading frame (ORF), and the open reading frame (ORF) has a nucleotide sequence as set forth in SEQ ID NO. 67, or SEQ ID NOs. 88-91, or, comprises a nucleotide sequence that is at least 80% identical to SEQ ID NO. 67, or SEQ ID NOs. 88-91. For example, the open reading frame (ORF) can, but is not limited to, comprise a nucleotide sequence that is at least 80%, 85%, 90%, 95% or 98% identical to SEQ ID NO. 67, or SEQ ID NOs. 88-91.

In some embodiments, the mRNA molecule comprises an open reading frame (ORF), and the open reading frame (ORF) has a nucleotide sequence as set forth in SEQ ID NO. 67, or, comprises a nucleotide sequence that is at least 80% identical to SEQ ID NO. 67. For example, the open reading frame (ORF) can, but is not limited to, comprise a nucleotide sequence that is at least 80%, 85%, 90%, 95% or 98% identical to SEQ ID NO. 67.

In some embodiments, the mRNA molecule comprises an open reading frame (ORF), and the open reading frame (ORF) has a nucleotide sequence as set forth in SEQ ID NO. 69, or, comprises a nucleotide sequence that is at least 80% identical to SEQ ID NO. 69. For example, the open reading frame (ORF) can, but is not limited to, comprise a nucleotide sequence that is at least 80%, 85%, 90%, 95% or 98% identical to SEQ ID NO. 69.

In some embodiments, the mRNA molecule comprises an open reading frame (ORF), and the open reading frame (ORF) has a nucleotide sequence as set forth in SEQ ID NO. 71, or SEQ ID NOs. 92-95, or, comprises a nucleotide sequence that is at least 80% identical to SEQ ID NO. 71, or SEQ ID NOs. 92-95. For example, the open reading frame (ORF) can, but is not limited to, comprise a nucleotide sequence that is at least 80%, 85%, 90%, 95% or 98% identical to SEQ ID NO. 71, or SEQ ID NOs. 92-95.

In some embodiments, the mRNA molecule comprises an open reading frame (ORF), and the open reading frame (ORF) has a nucleotide sequence as set forth in SEQ ID NO. 71, or, comprises a nucleotide sequence that is at least 80% identical to SEQ ID NO. 71. For example, the open reading frame (ORF) can, but is not limited to, comprise a nucleotide sequence that is at least 80%, 85%, 90%, 95% or 98% identical to SEQ ID NO. 71.

In some embodiments, the mRNA molecule comprises an open reading frame (ORF), and the open reading frame (ORF) has a nucleotide sequence as set forth in SEQ ID NO. 73, or, comprises a nucleotide sequence that is at least 80% identical to SEQ ID NO. 73. For example, the open reading frame (ORF) can, but is not limited to, comprise a nucleotide sequence that is at least 80%, 85%, 90%, 95% or 98% identical to SEQ ID NO. 73.

In some embodiments, the dimerization element has a nucleotide sequence as set forth in SEQ ID NO. 36 or SEQ ID NO. 42, or, has an optimized nucleotide sequence encoding the amino acid sequence as set forth in SEQ ID NOs. 1-2;
the trimerization element has a nucleotide sequence as set forth in SEQ ID NO. 37-40, SEQ ID NO. 43-46 or SEQ ID NO. 13, or, has an optimized nucleotide sequence encoding the amino acid sequence as set forth in SEQ ID NOs. 3-12;
the tetramerization element has a nucleotide sequence as set forth in SEQ ID NO. 41 or SEQ ID NO. 47, or, has an optimized nucleotide sequence encoding the amino acid sequence as set forth in SEQ ID NOs. 14-15.

Furthermore, in some embodiments, the multimerization element in the open reading frame (ORF) can also has a nucleotide sequence as set forth in SEQ ID NOs. 36-47 or SEQ ID NO. 13.

In some embodiments, nucleic acid molecules can optimize mRNA sequences by sequence optimization means to improve properties related to expression efficacy after *in vivo* administration: for example, to improve mRNA stability, increase translation efficacy in target tissues, reduce the number of truncated proteins expressed, improve the folding of expressed proteins or prevent their misfolding, reduce the toxicity of the expressed products, reduce cell death caused by expressed products, increase and/or reduce protein aggregation, thereby obtaining mRNA with improved properties. The purpose of sequence optimization also includes: optimizing the formulation and delivery characteristics of nucleic acid-based therapeutic agents while maintaining structural and functional integrity; overcoming the expression threshold; increasing the expression rate, half-life and/or protein concentration; optimizing protein localization; and avoiding adverse biological responses such as immune responses and/or degradation pathways. Sequence optimization means include: (1) codon optimization based on codon frequencies in specific organs and/or host organisms to ensure proper folding and appropriate expression; (2) adjustment of G/C content to increase mRNA stability or reduce secondary structure; (3) minimization of tandemly repeated codons or base runs that may impair gene construction or expression; (4) customization of transcription and translation control regions; and (5) reduction or elimination of problematic secondary structures within polynucleotides.

The "sequence identity" between two nucleotide sequences indicates the percentage of identical nucleotides between the sequences. The "sequence identity" between two amino acid sequences indicates the percentage of identical amino acids between the sequences.

The term "% identity" or similar terms refers to the percentage of nucleotides or amino acids which are identical in an optimal alignment between the sequences to be compared. Said percentage is purely statistical, and the differences between the two sequences may be but are not necessarily randomly distributed over the entire length of the sequences to be compared. Comparisons of two sequences are usually carried out by comparing the sequences, after optimal alignment, with respect to a segment or "window of comparison", in order to identify local regions of corresponding sequences.

In some embodiments, based on the mRNA open reading frame sequence provided, a person of ordinary skill in the art will be able to obtain the corresponding circular RNA open reading frame sequence, and prepare a complete sequence of the circular RNA capable of encoding the same amino acid sequence according to such literatures as CN202180048567.4. In some embodiments, based on the mRNA sequence provided, a person of ordinary skill in the art will be able to obtain the corresponding DNA sequence (e.g., uracil is converted to thymine). Similarly, based on the DNA sequence provided, a person of ordinary skill in the art will obtain the corresponding RNA sequence (e.g., thymine is converted to uracil). In some embodiments, based on the RNA or DNA sequence provided, a person of ordinary skill in the art will be able to obtain the corresponding amino acid sequence.

"Open reading frame (ORF)" according to the present disclosure refers to a continuous stretch of DNA or RNA that begins with a start codon (such as ATG or AUG) and ends with a stop codon (such as TAA, TAG or TGA, or UAA, UAG or UGA).

In some embodiments, the mRNA according to the present disclosure consists of a sequence comprising a 5' cap, a 5' UTR, an ORF, a 3' UTR and a 3' poly (A) tail sequentially from the 5' end to the 3' end.

The "5' untranslated region (UTR)" according to the present disclosure refers to a sequence located immediately upstream (i.e., 5') of the start codon (i.e., the first codon of the mRNA transcript translated by the ribosome) of an mRNA that does not encode a polypeptide. When an RNA transcript is produced, the 5'UTR may comprise a promoter sequence. Such promoter sequences are known in the art. The 5'UTR has an RNA sequence as set forth in one of SEQ ID NO. 16, SEQ ID NO. 17, or SEQ ID NO. 18, and alternatively, a 5'UTR sequence disclosed before the filing date of the present disclosure may also be incorporated into the present disclosure.

The "3' untranslated region (UTR)" according to the present disclosure refers to a sequence located downstream of the stop codon of an mRNA that does not encode a polypeptide. The 3'UTR has an RNA sequence as set forth in one of SEQ ID NO. 19, SEQ ID NO. 20 or SEQ ID NO. 21, and alternatively, a 3'UTR sequence disclosed before the filing date of the present disclosure may also be incorporated into the present disclosure.

The "poly(A) tail" according to the present disclosure refers to a sequence including multiple continuous adenosine monophosphates downstream from the 3'UTR of mRNA. The poly(A) tail may include 10 to 300 adenosine monophosphates. The poly (A) tail in a cell and/or *in vivo* may serve to protect the mRNA from an attack of an enzyme, and aid in termination of transcription, and/or export of the mRNA from the nucleus, and translation.

In some embodiments, the mRNA molecule includes a self-replicating mRNA molecule or a non-self-replicating mRNA molecule.

In some embodiments, the mRNA according to the present disclosure is a self-replicating mRNA, which carries a sequence capable of expressing RNA polymerase (RNA-dependent RNA polymerase, RdRP). Specifically, the contents regarding the sequence design of a self-replicating mRNA in Chinese patent CN202110424124.2 is incorporated into the present disclosure.

In some embodiments, the immunological composition comprises a delivery formulation.

In some embodiments, the delivery formulation comprises lipid nanoparticles or cationic liposomes.

In some embodiments, the components of the lipid nanoparticles include at least one of a protonatable cationic lipid, a structural lipid, a helper lipid, and a surfactant.

In some embodiments, the components of the lipid nanoparticles include, by weight, 40 to 60 parts of the protonable cationic lipid, 20 to 40 parts of the structural lipid, 10 to 30 parts of the helper lipid and 0.5 to 5 parts of the surfactant.

In some embodiments, the protonatable cationic lipid is selected from at least one of Dlin-MC3-DMA, DODMA, C12-200, and DlinDMA.

In some embodiments, the structural lipid includes cholesterol and/or a cholesterol derivative.

In some embodiments, the helper lipid includes at least one of DSPC, DOPE, DOPC, DOPG, and DOPS.

In some embodiments, the surfactant includes at least one of PEG-DMG, PEG-DSPE, and TPGS.

In some embodiments, the liposome nanoparticle comprises by molar percentage:
20% to 50% cationic lipid, for example, but not limited to 20%, 25%, 30%, 35%, 40%, 45% or 50%;
20% to 50% DOPG, for example, but not limited to 20%, 25%, 30%, 35%, 40%, 45% or 50%;
5% to 20% cholesterol, for example, but not limited to 5%, 10%, 15% or 20%; and
1% to 5% PEG-DMG, for example, but not limited to 1%, 2%, 3%, 4% or 5%.

In some embodiments, the liposome nanoparticle comprises by molar percentage:
20% to 50% cationic lipid, for example, but not limited to 20%, 25%, 30%, 35%, 40%, 45% or 50%;
20% to 50% DSCP, for example, but not limited to 20%, 25%, 30%, 35%, 40%, 45% or 50%;
5% to 20% cholesterol, for example, but not limited to 5%, 10%, 15% or 20%; and
1% to 5% PEG-DMG, for example, but not limited to 1%, 2%, 3%, 4% or 5%.

In some embodiments, the liposome nanoparticle comprises 50% Dlin-MC3-DMA, 10% DOPG, 38.5% cholesterol, and 1.5% PEG-DMG by molar percentage.

In some embodiments, the liposome nanoparticle comprises 50% Dlin-MC3-DMA, 10% DSCP, 38.5% cholesterol, and 1.5% PEG-DMG by molar percentage.

In some embodiments, the present disclosure provides a method for preparing an immunological composition as described in any of the aforementioned embodiments, the method comprising: the mRNA and a delivery formulation are mixed to form the immunological composition; the delivery formulation comprises lipid nanoparticles or cationic liposomes.

In some embodiments, the method for preparing an immunological composition is as follows: disslvoing the mRNA in a buffer to obtain an aqueous phase, measuring individual lipid components of the liposome nanoparticle and dissolving said components in an organic solvent to obtain an organic phase, mixing the aqueous phase and the organic phase, and then removing the organic phase to obtain the immunological composition.

In some embodiments, the volume ratio of the aqueous phase and the organic phase is 1:2-4, preferably 1:3.

In some embodiments, the buffer comprises citrate buffer or sodium acetate, preferably citrate buffer.

In some embodiments, the pH of the buffer is 3 to 7, preferably 4.

In some embodiments, the concentration of the mRNA in the aqueous phase is 0.05 mg/mL-0.5 mg/mL, preferably 0.1 mg/mL.

In some embodiments, the organic solvent is selected from C1 to C4 lower alcohols, preferably anhydrous ethanol.

In some embodiments, the concentration of the lipid component in the organic phase is 5 mg/mL-7 mg/mL, preferably 6 mg/mL.

In some embodiments, the aqueous phase and the organic phase are mixed by microfluidics, and the organic solvent is filtered using tangential flow. Preferably, the flow rate of the microfluidics is >3 ml/min, further preferably 12 mL/min.

In some embodiments, the method further comprises a concentration step after mixing, wherein the concentration step allows the final concentration of the mRNA to be from 50 µg/mL to 200 µg/mL, preferably 100 µg/mL.

In some embodiments, the lipid nanoparticle has a diameter of less than about 200 nm. In some embodiments, the lipid nanoparticle has a diameter of about 150 nm. In some embodiments, tthe lipid nanoparticle has a diameter of less than 100 nm. In some embodiments, the lipid nanoparticle has a diameter of about 55 nm to about 90 nm.

In some embodiments, the immunological composition comprising human respiratory syncytial virus (hRSV) antigen is a polypeptide immunological composition. The polypeptide immunological composition is an immunological composition prepared by chemical synthesis technology or genetic engineering technology according to the amino acid sequence of a known or predicted segment of an antigen epitope in the gene of human respiratory syncytial virus antigen. In some embodiments, the polypeptide immunological composition of the present disclosure is an immunological composition prepared by chemical synthesis technology according to the amino acid sequence of the pre-fusion hRSV F protein of the human respiratory syncytial virus, combined with artificial mutation and modification. In some embodiments, the polypeptide immunological composition of the present disclosure is obtained by artificial mutation and modification on the amino acid sequence of the pre-fusion hRSV F protein of the human respiratory syncytial virus, and then fermentation via genetic engineering technology (such as constructing genetically engineered bacteria by means of genetic engineering technology, etc.).

In some embodiments, the polypeptide immunological composition comprises a pre-fusion hRSV F protein, and the pre-fusion hRSV F protein has an amino acid sequence as set forth in SEQ ID NO. 62, SEQ ID NO. 64, SEQ ID NO. 66, SEQ ID NO. 68, SEQ ID NO. 70, SEQ ID NO. 72, SEQ ID NO. 76, or SEQ ID NO. 78; and/or
the pre-fusion hRSV F protein comprises an F2 sequence in which at least 1, 2, 3, 4, 5, 6, 7, 8, 9, 10, 11, or 12 amino acids between positions 98 to 109 are deleted sequentially or intermittently; and/or
the pre-fusion hRSV F protein comprises an RR1 sequence in which at least 1, 2, 3, 4, 5, 6, 7, or 8 amino acids between positions 137 to 144 are deleted sequentially or intermittently; and/or
the pre-fusion hRSV F protein comprises an RR2 sequence in which at least 1, 2, 3, 4, 5, 6, 7, 8, 9, 10, 11, 12, 13, 14, 15, 16, 17, 18, 19, 20, or 21 amino acids between positions 504 to 524 are deleted sequentially or intermittently; and/or
the pre-fusion hRSV F protein comprises a 525-529 sequence in which at least 1, 2, 3, 4, or 5 amino acids are deleted sequentially or intermittently; and/or
the pre-fusion hRSV F protein comprises a 551-574 sequence in which at least 1, 2, 3, 4, 5, 6, 7, 8, 9, 10, 11, 12, 13, or 14 amino acids are deleted sequentially or intermittently.

In some embodiments, the viral immunological composition described in the present disclosure carries the gene of human respiratory syncytial virus antigen hRSV F protein into the human body through harmless microorganism, eliciting an immune response by the body's immune system. Important viruses used in the induced cellular immunity assay include variants of vaccinia, polio virus, etc.

In some embodiments, the present disclosure provides an isolated mRNA, wherein the nucleic acid sequence of the coding region of the mRNA encodes a pre-fusion hRSV F protein, and the pre-fusion hRSV F protein has an amino acid sequence comprising a TM sequence and a multimerization element; preferably, the multimerization element has an amino acid sequence as set forth in SEQ ID NOs. 1-12, or SEQ ID NOs. 14-15; preferably, the multimerization element has an amino acid sequence as set forth in one of SEQ ID NO. 7 and SEQ ID NO. 12.

In some embodiments wherein the isolated mRNA is provided by the present disclosure, the pre-fusion hRSV F protein has the P27 sequence replaced with a GS sequence; preferably, the GS sequence comprises (GnS)m, (GGGGS)o, GGSGGGGSGG, GGSGGGGG, GSGSGSGS, (Gly)p, (EAAAK)q, such as SEQ ID NOs. 48-61, or SEQ ID NO. 74 (wherein n is 1, 2, 3, 4, 5, 6, 7, 8, 9, 10, 15 or 20; m is 1, 2, 3, 4, 5, 6, 7, 8, 9, 10, 15 or 20; o is an integer from 1 to 5; p is an integer from 1 to 40; q is an integer from 1 to 5); preferably, the GS sequence has an amino acid sequence as set forth in SEQ ID NO. 74.

In some embodiments wherein the isolated mRNA is provided by the present disclosure, the pre-fusion hRSV F protein has an amino acid sequence comprising at least one of an S46G substitution, an N67I substitution, an E92D substitution, a P102A substitution, an A149C substitution, an S155C substitution, an S190F substitution, a V207L substitution, an S290C substitution, an L373R substitution, an S215P substitution, an I379V substitution, an M447V substitution, a Y458C substitution, a K465Q substitution, a D486C substitution, and a D489C substitution.

In some embodiments wherein the isolated mRNA is provided by the present disclosure, the pre-fusion hRSV F protein has an amino acid sequence comprising at least one of an S155C substitution, an S190F substitution, a V207L substitution, an S290C substitution, a D486C substitution, and a D489C substitution.

In some embodiments wherein the isolated mRNA is provided by the present disclosure, the pre-fusion hRSV F protein has an amino acid sequence comprising at least one of an S155C substitution, an S190F substitution, a V207L substitution, and an S290C substitution, and/or the pre-fusion hRSV F protein has the P27 sequence replaced with a GS sequence.

In some embodiments wherein the isolated mRNA is provided by the present disclosure, the pre-fusion hRSV F protein has an amino acid sequence comprising an S155C substitution, an S190F substitution, a V207L substitution, and an S290C substitution; the pre-fusion hRSV F protein has the P27 sequence replaced with the GS sequence as set forth in SEQ ID NO. 74; the pre-fusion hRSV F protein comprises a multimerization element as set forth in one of SEQ ID NO. 7 and SEQ ID NO. 12.

In some embodiments wherein the isolated mRNA is provided by the present disclosure, the pre-fusion hRSV F protein has an amino acid sequence comprising an S155C substitution, an S190F substitution, a V207L substitution, and an S290C substitution; the pre-fusion hRSV F protein has the P27 sequence replaced with the GS sequence as set forth in SEQ ID NO. 74; the pre-fusion hRSV F protein comprises a multimerization element as set forth in one of SEQ ID NO. 7 and SEQ ID NO. 12; the amino acid sequence of the pre-fusion hRSV F protein also comprises at least one of an S46G substitution, an N67I substitution, an E92D substitution, a P102A substitution, an A149C substitution, an L373R substitution, an S215P substitution, an I379V substitution, an M447V substitution, a Y458C substitution, a K465Q substitution, a D486C substitution, and a D489C substitution.

In some embodiments wherein the isolated mRNA is provided by the present disclosure, the pre-fusion hRSV F protein has an amino acid sequence comprising at least one of an S155C substitution, an S190F substitution, a V207L substitution, an S290C substitution, a D486C substitution, and a D489C substitution; and/or the pre-fusion hRSV F protein has the P27 sequence replaced with a GS sequence.

In some embodiments wherein the isolated mRNA is provided by the present disclosure, the pre-fusion hRSV F protein has an amino acid sequence comprising an S155C substitution, an S190F substitution, a V207L substitution, an S290C substitution, a D486C substitution, and a D489C substitution; the pre-fusion hRSV F protein has the P27 sequence replaced with the GS sequence as set forth in SEQ ID NO. 74; and the pre-fusion hRSV F protein comprises a multimerization element as set forth in one of SEQ ID NO. 7 and SEQ ID NO. 12.

In some embodiments wherein the isolated mRNA is provided by the present disclosure, the pre-fusion hRSV F protein has an amino acid sequence comprising an S155C substitution, an S190F substitution, a V207L substitution, an S290C substitution, a D486C substitution, and a D489C substitution; the pre-fusion hRSV F protein has the P27 sequence replaced with the GS sequence as set forth in SEQ ID NO. 74; the pre-fusion hRSV F protein comprises a multimerization element as set forth in one of SEQ ID NO. 7 and SEQ ID NO. 12; and the amino acid sequence of the pre-fusion hRSV F protein also comprises at least one of an S46G substitution, an N67I substitution, an E92D substitution, a P102A substitution, an A149C substitution, an L373R substitution, an S215P substitution, an I379V substitution, an M447V substitution, a Y458C substitution, and a K465Q substitution.

In some embodiments wherein the isolated mRNA is provided by the present disclosure, the pre-fusion hRSV F protein variant comprises an amino acid sequence in which at least one of the amino acids in the F2 sequence, the RR1 sequence, the RR2 sequence, the 525-529 sequence or the 551-574 sequence is deleted sequentially or intermittently.

In some embodiments wherein the isolated mRNA is provided by the present disclosure, the pre-fusion hRSV F protein has an amino acid sequence as set forth in SEQ ID NO. 62, SEQ ID NO. 64, SEQ ID NO. 66, SEQ ID NO. 68, SEQ ID NO. 70, SEQ ID NO. 72, SEQ ID NO. 76, or SEQ ID NO. 78.

In some embodiments wherein the isolated mRNA is provided by the present disclosure, the pre-fusion hRSV F protein has an amino acid sequence as set forth in SEQ ID NO. 62, SEQ ID NO. 64, SEQ ID NO. 66, SEQ ID NO. 70, SEQ ID NO. 76, or SEQ ID NO. 78.

In some embodiments wherein the isolated mRNA is provided by the present disclosure, the pre-fusion hRSV F protein has an amino acid sequence as set forth in SEQ ID NO. 62, SEQ ID NO. 66, SEQ ID NO. 70, SEQ ID NO. 76, or SEQ ID NO. 78.

In some embodiments wherein the isolated mRNA is provided by the present disclosure, the multimerization element of the pre-fusion hRSV F protein can also be selected from the amino acid sequences as set forth in SEQ ID NOs. 1-12 and SEQ ID NOs. 14-15.

In some embodiments, the isolated mRNA comprises an open reading frame (ORF), and the open reading frame (ORF) has a nucleotide sequence as set forth in SEQ ID NO. 63, SEQ ID NO. 65, SEQ ID NO. 67, SEQ ID NO. 69, SEQ ID NO. 71, SEQ ID NO. 73, SEQ ID NO. 77, SEQ ID NO. 79, or SEQ ID NOs. 84-103, or, comprises a nucleotide sequence that is at least 80% identical to SEQ ID NO. 63, SEQ ID NO. 77, SEQ ID NO. 79, SEQ ID NOs. 84-87, or SEQ ID NOs. 96-103. For example, the open reading frame (ORF) can, but is not limited to, comprise a nucleotide sequence that is at least 80%, 85%, 90%, 95% or 98% identical to SEQ ID NO. 63, SEQ ID NO. 77, SEQ ID NO. 79, SEQ ID NOs. 84-87, or SEQ ID NOs. 96-103.

In some embodiments, the isolated mRNA comprises an open reading frame (ORF), and the open reading frame (ORF) has a nucleotide sequence as set forth in SEQ ID NO. 63, SEQ ID NO. 67, SEQ ID NO. 71, SEQ ID NO. 77, SEQ ID NO. 79, or SEQ ID NOs. 84-103, or, comprises a nucleotide sequence that is at least 80% identical to SEQ ID NO. 63, SEQ ID NO. 67, SEQ ID NO. 71, SEQ ID NO. 77, SEQ ID NO. 79, or SEQ ID NOs. 84-103. For example, the open reading frame (ORF) can, but is not limited to, comprise a nucleotide sequence that is at least 80%, 85%, 90%, 95% or 98% identical to SEQ ID NO. 63, SEQ ID NO. 67, SEQ ID NO. 71, SEQ ID NO. 77, SEQ ID NO. 79, or SEQ ID NOs. 84-103.

In some embodiments, the isolated mRNA comprises an open reading frame (ORF), and the open reading frame (ORF) has a nucleotide sequence as set forth in SEQ ID NO. 63, SEQ ID NO. 71, SEQ ID NO. 77, SEQ ID NO. 79, SEQ ID NOs. 84-87, or SEQ ID NOs. 92-103, or, comprises a nucleotide sequence that is at least 80% identical to SEQ ID NO. 63, SEQ ID NO. 71, SEQ ID NO. 77, SEQ ID NO. 79, SEQ ID NOs. 84-87, or SEQ ID NOs. 92~103. For example, the open reading frame (ORF) can, but is not limited to, comprise a nucleotide sequence that is at least 80%, 85%, 90%, 95% or 98% identical to SEQ ID NO. 63, SEQ ID NO. 71, SEQ ID NO. 77, SEQ ID NO. 79, SEQ ID NOs. 84-87, or SEQ ID NOs. 92-103.

In some embodiments, the isolated mRNA comprises an open reading frame (ORF), and the open reading frame (ORF) has a nucleotide sequence as set forth in SEQ ID NO. 63, SEQ ID NO. 77, SEQ ID NO. 79, SEQ ID NOs. 84-87, or SEQ ID NOs. 96~103, or, comprises a nucleotide sequence that is at least 80% identical to SEQ ID NO. 63, SEQ ID NO. 77, SEQ ID NO. 79, SEQ ID NOs. 84-87, or SEQ ID NOs. 96~103. For example, the open reading frame (ORF) can, but is not limited to, comprise a nucleotide sequence that is at least 80%, 85%, 90%, 95% or 98% identical to SEQ ID NO. 63, SEQ ID NO. 77, SEQ ID NO. 79, SEQ ID NOs. 84-87, or SEQ ID NOs. 96~103.

In some embodiments, the isolated mRNA comprises an open reading frame (ORF), and the open reading frame (ORF) has a nucleotide sequence as set forth in SEQ ID NO. 63, or SEQ ID NOs. 84-87, or, comprises a nucleotide sequence that is at least 80% identical to SEQ ID NO. 63, or SEQ ID NOs. 84-87. For example, the open reading frame (ORF) can, but is not limited to, comprise a nucleotide sequence that is at least 80%, 85%, 90%, 95% or 98% identical to SEQ ID NO. 63, or SEQ ID NOs. 84-87.

In some embodiments, the isolated mRNA comprises an open reading frame (ORF), and the open reading frame (ORF) has a nucleotide sequence as set forth in SEQ ID NO. 63, or, comprises a nucleotide sequence that is at least 80% identical to SEQ ID NO. 63. For example, the open reading frame (ORF) can, but is not limited to, comprise a nucleotide sequence that is at least 80%, 85%, 90%, 95% or 98% identical to SEQ ID NO. 63.

In some embodiments, the isolated mRNA comprises an open reading frame (ORF), and the open reading frame (ORF) has a nucleotide sequence as set forth in SEQ ID NO. 77, or SEQ ID NOs. 96-99, or, comprises a nucleotide sequence that is at least 80% identical to SEQ ID NO. 77, or SEQ ID NOs. 96-99. For example, the open reading frame (ORF) can, but is not limited to, comprise a nucleotide sequence that is at least 80%, 85%, 90%, 95% or 98% identical to SEQ ID NO. 77, or SEQ ID NOs. 96-99.

In some embodiments, the isolated mRNA comprises an open reading frame (ORF), and the open reading frame (ORF) has a nucleotide sequence as set forth in SEQ ID NO. 77, or, comprises a nucleotide sequence that is at least 80% identical to SEQ ID NO. 77. For example, the open reading frame (ORF) can, but is not limited to, comprise a nucleotide sequence that is at least 80%, 85%, 90%, 95% or 98% identical to SEQ ID NO. 77.

In some embodiments, the isolated mRNA comprises an open reading frame (ORF), and the open reading frame (ORF) has a nucleotide sequence as set forth in SEQ ID NO. 79, or SEQ ID NOs. 100-103, or, comprises a nucleotide sequence that is at least 80% identical to SEQ ID NO. 79, or SEQ ID NOs. 100-103. For example, the open reading frame (ORF) can, but is not limited to, comprise a nucleotide sequence that is at least 80%, 85%, 90%, 95% or 98% identical to SEQ ID NO. 79, or SEQ ID NOs. 100-103.

In some embodiments, the isolated mRNA comprises an open reading frame (ORF), and the open reading frame (ORF) has a nucleotide sequence as set forth in SEQ ID NO. 79, or, comprises a nucleotide sequence that is at least 80% identical to SEQ ID NO. 79. For example, the open reading frame (ORF) can, but is not limited to, comprise a nucleotide sequence that is at least 80%, 85%, 90%, 95% or 98% identical to SEQ ID NO. 79.

In some embodiments, the isolated mRNA comprises an open reading frame (ORF), and the open reading frame (ORF) has a nucleotide sequence as set forth in SEQ ID NO. 65, or, comprises a nucleotide sequence that is at least 80% identical to SEQ ID NO. 65. For example, the open reading frame (ORF) can, but is not limited to, comprise a nucleotide sequence that is at least 80%, 85%, 90%, 95% or 98% identical to SEQ ID NO. 65.

In some embodiments, the isolated mRNA comprises an open reading frame (ORF), and the open reading frame (ORF) has a nucleotide sequence as set forth in SEQ ID NO. 67, or SEQ ID NOs. 88-91, or, comprises a nucleotide sequence that is at least 80% identical to SEQ ID NO. 67, or SEQ ID NOs. 88-91. For example, the open reading frame (ORF) can, but is not limited to, comprise a nucleotide sequence that is at least 80%, 85%, 90%, 95% or 98% identical to SEQ ID NO. 67, or SEQ ID NOs. 88-91.

In some embodiments, the isolated mRNA comprises an open reading frame (ORF), and the open reading frame (ORF) has a nucleotide sequence as set forth in SEQ ID NO. 67, or comprises a nucleotide sequence that is at least 80% identical to SEQ ID NO. 67. For example, the open reading frame (ORF) can, but is not limited to, comprise a nucleotide sequence that is at least 80%, 85%, 90%, 95% or 98% identical to SEQ ID NO. 67.

In some embodiments, the isolated mRNA comprises an open reading frame (ORF), and the open reading frame (ORF) has a nucleotide sequence as set forth in SEQ ID NO. 69, or, comprises a nucleotide sequence that is at least 80% identical to SEQ ID NO. 69. For example, the open reading frame (ORF) can, but is not limited to, comprise a nucleotide sequence that is at least 80%, 85%, 90%, 95% or 98% identical to SEQ ID NO. 69.

In some embodiments, the isolated mRNA comprises an open reading frame (ORF), and the open reading frame (ORF) has a nucleotide sequence as set forth in SEQ ID NO. 71, or SEQ ID NOs. 92~95, or, comprises a nucleotide sequence that is at least 80% identical to SEQ ID NO. 71, or SEQ ID NOs. 92~95. For example, the open reading frame (ORF) can, but is not limited to, comprise a nucleotide sequence that is at least 80%, 85%, 90%, 95% or 98% identical to SEQ ID NO. 71, or SEQ ID NOs. 92~95.

In some embodiments, the isolated mRNA comprises an open reading frame (ORF), and the open reading frame (ORF) has a nucleotide sequence as set forth in SEQ ID NO. 71, or, comprises a nucleotide sequence that is at least 80% identical to SEQ ID NO. 71. For example, the open reading frame (ORF) can, but is not limited to, comprise a nucleotide sequence that is at least 80%, 85%, 90%, 95% or 98% identical to SEQ ID NO. 71.

In some embodiments, the isolated mRNA comprises an open reading frame (ORF), and the open reading frame (ORF) has a nucleotide sequence as set forth in SEQ ID NO. 73, or, comprises a nucleotide sequence that is at least 80% identical to SEQ ID NO. 73. For example, the open reading frame (ORF) can, but is not limited to, comprise a nucleotide sequence that is at least 80%, 85%, 90%, 95% or 98% identical to SEQ ID NO. 73.

Furthermore, in some embodiments, the nucleotide sequence of the multimerization element in the open reading frame (ORF) of the isolated mRNA can also be as set forth in SEQ ID NOs. 36-47 or SEQ ID NO. 13.

In some embodiments, the present disclosure provides an isolated DNA, wherein the DNA is obtained by reverse transcription of the isolated mRNA or mRNA molecule nucleic acid as described in any of the aforementioned embodiments, or the DNA is a sequence that can be transcribed into the isolated mRNA or mRNA molecule.

In some embodiments, the isolated DNA is an isolated stranded DNA or an isolated circular DNA.

In some embodiments, the present disclosure provides a biological material, comprising any one of an expression cassette, a vector, an engineered bacterium or a cell line, wherein the biological material contains or expresses the isolated mRNA as described in any of the aforementioned embodiments or the isolated DNA as described in any of the aforementioned embodiments.

In some embodiments, the present disclosure provides a method comprising administering to a subject an effective amount of at least one of the immunological composition described in any of the aforementioned embodiments, the isolated mRNA described in any of the aforementioned embodiments, the isolated DNA described in any of the aforementioned embodiments, or the biological material described in any of the aforementioned embodiments, to induce a neutralizing antibody response against human syncytial virus in the subject.

In some embodiments, the method comprises administering to the subject at least twice an effective amount of at least one of the immunological composition described in any of the aforementioned embodiments, the isolated mRNA described in any of the aforementioned embodiments, the isolated DNA described in any of the aforementioned embodiments, or the biological material described in any of the aforementioned embodiments.

In some embodiments, the time interval between the first administration and the second administration in the at least two administrations is no less than 14 days.

In some embodiments, the subject is immunocompromised; in some embodiments, the subject is no more than 5 years old or no less than 65 years old.

In some embodiments, the effective amount described in the present disclosure is as low as 40 µg, 30 µg, 25 µg, 20 µg, 15 µg, 10 µg, 5 µg, 3 µg or 1 µg of the immunological composition described in any of the aforementioned embodiments, the isolated mRNA described in any of the aforementioned embodiments, the isolated DNA described in any of the aforementioned embodiments, or the biological material described in any of the aforementioned embodiments.

### Example 1: Process for preparing LNP from RNA

A lipid nanoparticle comprising RNA encoding a respiratory syncytial virus antigen, wherein the lipid nanoparticle comprises, by molar percentage, 50% of Dlin-MC3-DMA, 20% of DOPG, 29% of cholesterol and 1% of PEG-DMG.

The preparation method is as follows:
(a) The RNA was dissolved in a citrate buffer at pH 4, and the concentration was adjusted to 0.1 mg/ml to obtain an aqueous phase.
(b) Dlin-MC3-DMA, DOPG, cholesterol, and PEG-DMG were dissolved in absolute ethanol according to the formula amount, and the concentration of the lipid component in the organic phase was adjusted to 6 mg/mL to obtain an organic phase.
(c) The aqueous phase of step (a) and the organic phase of step (b) were mixed at a volume ratio of 1:3 using a microfluidic device at a flow rate of 12 mL/min. The mixed solution was immediately diluted 100 times with PBS solution at pH 7.4, and the ethanol component was removed from the solution by tangential flow filtration (TFF). The solution was then concentrated until the concentration of mRNA in the system was 55 µg/ml, to obtain a lipid nanoparticle containing RNA encoding the respiratory syncytial virus antigen.

### Example 2

The formulations with different vaccine vectors were examined by *in vivo* fluorescence imaging technology, using luciferase as a reporter gene (as shown in Table 1 below).

**Table 1**

| No. | Lipid Nanoparticle Formulation | | Particle Size (nm) | Surface potential (mV) | Encapsulation rate (%) |
|---|---|---|---|---|---|
| | Components | Components in molar ratio | | | |
| 1 | MC3/cholesterol/DSPC/PEG-DMG | 50:38.5:10:1.5 | 82 | -2.1 | 75-80 |
| 2 | DODMA/cholesterol/DOPE/PEG-DSPE | 40:40:15:5 | 20 | -3.2 | 79-82 |

"MC3" refers to Dlin-MC3-DMA, "+": luciferase expression was detected in the mouse after administration by the Fluorescence *in Vivo* Small Animal Imaging System) The efficiency of delivering mRNA encoding the luciferase gene in the mouse, the physical and chemical indicators of different composite preparations (see Example 1 for preparation method) were tested, and the results are shown in Table 1. The study found that increasing the mass ratio of lipid to mRNA is beneficial to increasing the encapsulation rate of mRNA in the lipid nanoparticle so that it has higher stability. In addition, moderately increasing the content of polyethylene glycol (PEG) in the formula is beneficial to improving the expression efficiency of mRNA in vivo. Therefore, considering both the mRNA encapsulation rate and the efficiency of mRNA delivery *in vivo,* formulas 3 and 4 were selected for subsequent mRNA vaccine research.

### Example 3

**Table 2**

| No. | Components in molar ratio: MC3/cholesterol/D SPC/PEG-DMG | Lipid/mRNA | Particle Size (nm) | Surface potential (mV) | Encapsula tion rate (%) | luciferase signal visualized by mouse imaging in vivo |
|---|---|---|---|---|---|---|
| I | 50:38.5:10:1.5 | 10 | 80 | -3 | 75-80 | N/A |
| 2 | 50:40:7:3 | 10 | 82 | -4 | 79-82 | N/A |
| 3 | 50:38.5:10:1.5 | 20 | 76 | -3.2 | 82-90 | +++ |
| 4 | 50:40:7:3 | 20 | 70 | -4.1 | 85-91 | +++ |
| 5 | 40:50:7:3 | 20 | 65 | -2.5 | 85-91 | ++ |

The ability of cationic lipid nanoparticles of different formulations to encapsulate mRNA encoding luciferase and the particle size of the formed nanoparticles are shown in Table 2. Several formulations can compress luciferase mRNA into nanoparticles with a particle size of less than 100 nm and a net neutral surface potential, and can encapsulate at least 50% of mRNA; therefore, they all have an *in vivo* delivery effect. "MC3" refers to Dlin-MC3-DMA.

### Example 4

BALB/c mice (female, 5-6 weeks old, average body weight 20-25 g, purchased from Zhuhai Baishitong Biotechnology Co., Ltd.) were selected for vaccine immunogenicity evaluation.

The experimental animals were randomly divided into groups, with 5 mice in each group. The immunization dose was 15 µg/mouse, and the same dose of booster immunization was given 2 weeks after vaccination. Twelve days after the second immunization, the indicators for cellular immune response and humoral immune response were determined.

The mouse lymphocytes were separated according to the following procedure: the mice were sacrificed by cervical dislocation and soaked in 75% ethanol; the mouse spleen was removed in a clean bench; 4mL-5mL mouse lymphocyte separation medium (restored to room temperature and shaken before use) was placed into a 35mm culture dish; ground; the separation medium in which spleen cells were suspended was immediately transferred to a 15mL centrifuge tube, and centrifuged at room temperature at 800g for 30 minutes in a horizontal rotor. After the centrifugation, the lymphocyte layer was aspirated off, 10mL RPMI 1640 culture medium was added, and the tube was inverted for washing. The cells were collected by centrifugation at room temperature. The experimental process can refer to Figure 3.

### 1. Determination of RSV-F protein-specific IgG antibodies in mouse serum (humoral immunity) by ELISA

The wild-type hRSV F protein (His Tag) (2 mg/mL) diluted to 2 µg/mL was added to the multi-well plate (100 µL per well) with washing solution and coated overnight. The wells were washed and blocked with 2% BSA solution.

All serum samples to be tested were diluted with washing solution in a 10-fold gradient starting from 1: 100 to 1:1,000,000. The negative control samples were kept consistent with the serum samples to be tested and were diluted by the same fold, with a total of 6 gradient samples.

The prepared serum sample solution was added to the enzyme-labeled multi-well plate, incubated and washed. The diluted enzyme-labeled antibody solution was added, incubated and washed. The wells were subject to color development, and the OD value at 450 nm/630 nm was measured on the plate reader.

The cutoff value was 2.1 times the arithmetic mean of the signal from the negative serum sample.

### 2. Detection of TNFα+ CD4+ and TNF-α+ CD8+ T cells (cellular immunity) by flow cytometry

The separated mouse lymphocytes were stimulated with peptide library, and cultured in a 37°C, 5% CO₂ incubator for 72 h. The cells were stimulated overnight with BD GolgiPlµgTM (containing Brefeldin A) protein transport inhibitor (containing BSA) solution. The mouse lymphocyte suspension was centrifuged and the pellet was retained.

The mouse lymphocyte pellet was resuspended, and specific monoclonal fluorescent antibodies, such as CD4, CD8, etc. were added. The cells were incubated and centrifuged, and the mouse lymphocyte pellet was retained.

The fixation/permeabilization solution was added, incubated and centrifuged to obtain mouse lymphocyte pellet. Buffer was added to the mouse lymphocyte pellet, mixed well and centrifuged to obtain mouse lymphocyte pellet.

The mouse lymphocyte pellet was resuspended with staining buffer, and cytokine fluorescent antibodies, such as IFN-γ, TNF-α, etc., were added. The cells were incubated in the dark and centrifuged, and the mouse lymphocyte pellet was retained. Then the mouse lymphocyte pellet was resuspended with staining buffer and tested on flow cytometer.

The expression of TNF-α in CD3+/CD4+ and CD3+/CD8+ T lymphocytes was detected by flow cytometry.

### 3. Detection of IFN-γ+ CD4+ and IFN-γ+ CD8+ T cells (cellular immunity) by flow cytometry

The separated mouse lymphocytes were stimulated with peptide library, and cultured in a 37°C, 5% CO₂ incubator for 72 h. The cells were stimulated overnight with BD GolgiPlµgTM (containing Brefeldin A) protein transport inhibitor (containing BSA) solution. The mouse lymphocyte suspension was centrifuged and the pellet was retained.

The mouse lymphocyte pellet was resuspended, and specific monoclonal fluorescent antibodies, such as CD4, CD8, etc. were added. The cells were incubated and centrifuged, and the mouse lymphocyte pellet was retained.

The fixation/permeabilization solution was added, incubated and centrifuged to obtain mouse lymphocyte pellet. Buffer was added to the mouse lymphocyte pellet, mixed well and centrifuged to obtain mouse lymphocyte pellet.

The mouse lymphocyte pellet was resuspended with staining buffer, and cytokine fluorescent antibodies, such as IFN-γ, TNF-α, etc., were added. The cells were incubated in the dark and the mouse lymphocyte pellet was retained. Then the mouse lymphocyte pellet was resuspended with staining buffer and tested on flow cytometer.

The expression of IFN-γ in CD3+/CD4+ and CD3+/CD8+ T lymphocytes was detected by flow cytometry.

### 4. Detection of IFN-γ and IL2 in cell culture supernatant (cellular immunity) by ELISA

The separated mouse lymphocytes were stimulated with peptide library, and cultured in a 37°C, 5% CO2 incubator for 72 h. The mouse lymphocyte suspension was centrifuged to obtain the supernatant.

The IFN-γ and IL2 contents in the supernatant were determined using the mouse IL-2 ELISA kit (purchased from Xinbosheng Biotechnology Co., Ltd.) and mouse IFN-γ ELISA kit (purchased from Xinbosheng Biotechnology Co., Ltd.).

### 5. Detection of IL4 and IL10 in cell culture supernatant (cellular immunity) by ELISA

The separated mouse lymphocytes were stimulated with peptide library, and cultured in a 37°C, 5% CO₂ incubator for 72 h. The mouse lymphocyte suspension was centrifuged to obtain the supernatant. The IL4 and IL10 contents in the supernatant were determined using the mouse IL-4 ELISA kit (purchased from Xinbosheng Biotechnology Co., Ltd.) and mouse IL-10 ELISA kit (purchased from Xinbosheng Biotechnology Co., Ltd.).

### 6. Neutralizing activity of serum

The neutralizing activity of serum in the immunological composition group was determined as follows: HEp-2 cells were digested; the cell density was adjusted; the cells were inoculated into a 96-well plate and cultured overnight.

Each mouse serum dilution and virus dilution were added to the positive wells of the multi-well plate, and the virus dilution was added to the negative wells. Then, the diluted virus was added to the positive wells and negative wells of the multi-well plate, and the mixture was incubated for neutralization.

The neutralization products in the positive wells and negative wells of the multi-well plate were added to the multi-well plate containing the completely digested HEp-2 cells, respectively, and cultured. The neutralization products were aspirated off, DMEM + 2% FBS culture medium was added to each well and the culturing process continued.

The cell supernatant was discarded, and 4% paraformaldehyde was added to each well to fix the cells. The cells were then washed with PBS washing solution, and the cells were blocked with a 1:1 mixture of 4% BSA and 0.2% triton. The blocking solution was discarded, and the F6-6-488 antibody dilution solution was added, incubated and washed. The multi-well plate was spin-dried and read using a CTL device.

### Example 5

The nucleotide sequences described in the following table are mRNA sequences. The mRNA sequences described in the following table are formulated into lipid nanoparticles containing RNAs encoding respiratory syncytial virus antigens according to the method described in Example 1.

**Table 3**

| Sample Name | Amino acid sequence | ORF nucleotide sequence | Sample Name | Amino acid sequence | ORF nucleotide sequence |
|---|---|---|---|---|---|
| Wt | SEQ ID NO. 24 | SEQ ID NO. 25 | DS-Cav1 | SEQ ID NO. 26 | SEQ ID NO. 27 |
| F7-5 | SEQ ID NO. 7**0** | SEQ ID NO. 71 | DS-Cav1+Tric | SEQ ID NO. 32 | SEQ ID NO. 33 |

The mRNA in the mRNA immunological composition encoding respiratory syncytial virus antigen comprises, in addition to the reading frame sequence in the above table, a 5' cap (m7Gppp (5')), a 5' UTR (as set forth in SEQ ID NO. 16), a 3' UTR (as set forth in SEQ ID NO. 19) and a 3' polyA tail of 100 adenine nucleotides; and the uracil of the mRNA is replaced with a 5' pseudouracil.

In addition to the above lipid nanoparticles containing RNAs encoding respiratory syncytial virus antigens, an F protein (F-Protein) experimental group and a PBS blank control group were introduced in the experiment.

Sample Wt is an RNA lipid nanoparticle containing an amino acid sequence encoding the wild-type RSV F protein;
the amino acid sequence of the RSV F protein variant of sample F7-5 is based on the RSV F protein wild-type, with S155C/S190F/V207L/S290C substitutions, wherein Fibritin Dominain is added to the C-terminus (the amino acid sequence is set forth in SEQ ID NO. 7);
the amino acid sequence of the RSV F protein variant of sample DS-Cav1 is based on the RSV F protein wild-type, with S155C/S190F/V207L/S290C substitutions;
the amino acid sequence of the RSV F protein variant of sample DS-Cav1+Tric is based on the RSV F protein wild-type, with S155C/S190F/V207L/S290C substitutions, wherein the TM sequence is deleted, and Fibritin Dominain is added to the C-terminus (the amino acid sequence is set forth in SEQ ID NO. 7).

The vaccine immunogenicity of the prepared lipid nanoparticles of RNAs encoding respiratory syncytial virus antigens was evaluated according to the method of Example 4.

### 1. Detection of TNFα+ CD4+ and TNF-α+ CD8+ T cells by flow cytometry

The results are shown in Figure 4. The results showed that there was no significant difference in TNF-α+ CD8+ between the mRNA immunological composition groups and the F-protein immunological composition group (P>0.01). There was no significant difference in TNF-α+ CD4+ between the mRNA immunological composition groups and the F-protein immunological composition group (P>0.01). Compared with the PBS group, both the mRNA immunological composition groups and the F-protein immunological composition group could significantly stimulate Th1 type cellular immune response (P<0.01).

### 2. Determination of IFN-γ and IL4 in cell culture supernatant by ELISA

The IFN-γ determination results are shown in Figure 5. The results showed that there was no significant difference in IFN-γ between the mRNA immunological composition groups and the F-protein immunological composition group (P>0.01). Compared with the PBS group, both the mRNA immunological composition groups and the F-protein immunological composition group could significantly stimulate Th1 type cellular immune responses.

The IL4 determination results are shown in Figure 5. The results showed that: except for the sample DS-Cav1 immunological composition group, the IL-4 of the other mRNA immunological composition groups was significantly higher than that of the F-protein immunological composition group (P<0.01). Compared with the PBS group, both the mRNA immunological composition groups and the F-protein immunological composition group could significantly stimulate Th2-type cellular immune responses.

### 3. Neutralizing activity of serum

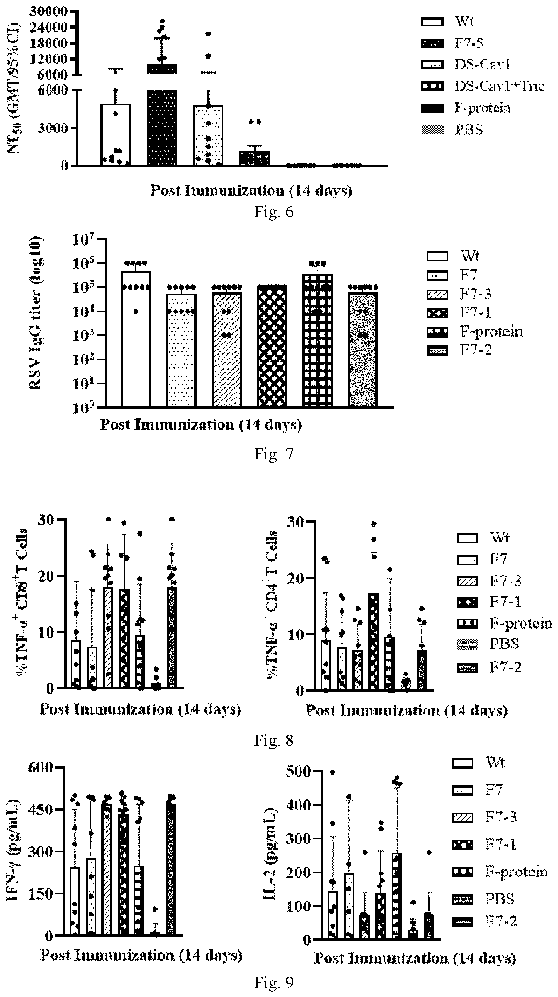

14 days after the second immunization, the RSV neutralizing antibody titer in the mouse serum was measured by the plaque reduction assay directed to the RSV live virus and the results are shown in Figure 6. Compared with the RSV F protein wild-type mRNA immunological composition group and other RSV F protein mutant mRNA immunological composition groups, the geometric mean titer of the RSV neutralizing antibody in the F7-5 immunological composition group was 10119.3, which was significantly higher than other mRNA immunological composition groups, F-protein immunological composition group and PBS control group (P<0.01).

In this Example, regarding the RSV F protein mutant mRNA immunological composition group based on the RSV F protein wild type with S155C/S190F/V207L/S290C substitutions, when the TM sequence is retained and multimerization elements are added (such as sample F7-5), the RSV neutralizing antibody titer in the mouse serum is significantly higher than that of only retaining the TM sequence (such as sample DS-Cav1) or only adding the multimerization element (such as sample DS-Cav1+Tric).

### Example 6

Lipid nanoparticles containing RNAs encoding respiratory syncytial virus antigens were prepared according to the method described in Example 2, which were designated as samples Wt, F7, F7-3, F7-1, and F7-2, respectively.

The sequence of the RNA open reading frame in the lipid nanoparticle containing RNA encoding the respiratory syncytial virus antigen according to sample Wt is set forth in SEQ ID NO. 25. The encoded respiratory syncytial virus antigen is RSV F protein wild type (The amino acid sequence is set forth in SEQ ID NO. 24).

The sequence of the RNA open reading frame in the lipid nanoparticle containing RNA encoding the respiratory syncytial virus antigen according to sample F7 is set forth in SEQ ID NO. 23, and the encoded respiratory syncytial virus antigen is a RSV F protein mutant (the amino acid sequence is set forth in SEQ ID NO. 22). The encoded amino acid sequence of the RSV F protein variant according to sample F7 is based on the RSV F protein wild type, with S155C/S190F/V207L/S290C/D486C/D489C substitutions, wherein the P27 sequence is replaced by the GS sequence as set forth in SEQ ID NO. 74, the TM sequence is deleted, and Fibritin Dominain is added to the C-terminus (the amino acid sequence is set forth in SEQ ID NO. 7).

The sequence of the RNA open reading frame in the lipid nanoparticle containing RNA encoding the respiratory syncytial virus antigen according to sample F7-3 is set forth in SEQ ID NO. 29, and the encoded respiratory syncytial virus antigen is a RSV F protein mutant (the amino acid sequence is set forth in SEQ ID NO. 28). The encoded amino acid sequence of the RSV F protein variant according to sample F7-3 is based on the RSV F protein wild type, with S155C/S190F/V207L/S290C/D486C/D489C substitutions, wherein the P27 sequence is replaced by the GS sequence as set forth in SEQ ID NO. 28, the TM sequence is deleted, and GCN4 Dominain is added to the C-terminus (the amino acid sequence is set forth in SEQ ID NO. 12).

The sequence of the RNA open reading frame in the lipid nanoparticle containing RNA encoding the respiratory syncytial virus antigen according to sample F7-1 is set forth in SEQ ID NO. 63, and the encoded respiratory syncytial virus antigen is a RSV F protein mutant (the amino acid sequence is set forth in SEQ ID NO. 62). The encoded amino acid sequence of the RSV F protein variant according to sample F7-1 is based on the RSV F protein wild type, with S155C/S190F/V207L/S290C/D486C/D489C substitutions, wherein the P27 sequence is replaced by the GS sequence as set forth in SEQ ID NO. 74, and Fibritin Dominain is added to the C-terminus (the amino acid sequence is set forth in SEQ ID NO. 7).

The sequence of the RNA open reading frame in the lipid nanoparticle containing RNA encoding the respiratory syncytial virus antigen according to sample F7-2 is set forth in SEQ ID NO. 65, and the encoded respiratory syncytial virus antigen is a RSV F protein mutant (the amino acid sequence is set forth in SEQ ID NO. 64). The encoded amino acid sequence of the RSV F protein variant according to sample F7-2 is based on the RSV F protein wild type, with S155C/S190F/V207L/S290C/D486C/D489C substitutions, wherein the P27 sequence is replaced by the GS sequence as set forth in SEQ ID NO. 74.

The mRNA in the mRNA immunological composition encoding the respiratory syncytial virus antigen comprises, in addition to the above-mentioned reading frame sequence, a 5' cap (m7Gppp (5')), a 5' UTR (as set forth in SEQ ID NO. 16), a 3' UTR (as set forth in SEQ ID NO. 19) and a 3' polyA tail of 100 adenine nucleotides; and the uracil of the mRNA is replaced with a 5' pseudouracil.

In addition to the above lipid nanoparticles containing RNAs encoding respiratory syncytial virus antigens, F protein (purchased from Sino Biological) experimental group and PBS blank control group were introduced in the experiment.

The prepared lipid nanoparticles encoding RNAs encoding respiratory syncytial virus antigens were evaluated for vaccine immunogenicity according to the method of Example 4.

### (1) Determination of RSV-F protein specific IgG in serum by ELISA

14 days after the second immunization, RSV-F protein specific IgG antibody in mouse serum was determined by ELISA. The serum was diluted from 100 times to 10 million times, and OD450 was measured by indirect ELISA. Statistics on the specific IgG antibody titer of mice in the RSV F protein mRNA immunological composition group showed (see Figure 7) that there was no significant difference in the IgG antibody titer between the RSV F protein wild-type mRNA immunological composition group (Wt) or the RSV F protein mutant mRNA immunological composition groups (F7, F7-3, F7-1 and F7-2), and the F-protein immunological composition group.

### (2) Detection of TNFα+ CD4+ and TNF-α+ CD8+ T cells by flow cytometry

14 days after immunization, mouse spleen lymphocytes were isolated, and the expression of TNF-α in CD3+/CD4+ and CD3+/CD8+ T lymphocytes was detected by flow cytometry. The results are shown in Figure 8.

The results showed that TNF-α+ CD8+ in the F7-3 immunological composition group and F7-2 immunological composition group was significantly higher than that in the F-protein immunological composition group (P<0.01). There was no significant difference in TNF-α+ CD4+ in the mRNA immunological composition group compared with the F-protein immunological composition group (P>0.01). TNF- α+ CD8+/CD4+ in the RSV F protein wild-type mRNA immunological composition group (Wt), RSV F protein mutant mRNA immunological composition group (F7, F7-3, F7-1 and F7-2) and F-protein immunological composition group was significantly higher than that in the PBS group (P<0.01).

The above results showed that RSV F protein wild-type mRNA immunological composition group (Wt), RSV F protein mutant mRNA immunological composition groups (F7, F7-3, F7-1 and F7-2) and F-protein immunological composition group can significantly stimulate the Th1 type cellular immune response in mice.

### (3) Determination of IFN-γ and IL-2 in spleen T lymphocyte culture supernatant by ELISA

14 days after immunization, the expression of IFN-γ and IL-2 in the lymphocyte supernatant was determined by ELISA. The results are shown in Figure 9.

The results showed that IFN-γ in the F7-3, F7-1 immunological composition groups and F7-2 immunological composition group was significantly higher than that in the F-protein immunological composition group (P<0.01). IL-2 in the F7-3 immunological composition group and F7-2 immunological composition group was significantly higher than that in the F-protein immunological composition group (P<0.01). IFN- γ and IL-2 in the RSV F protein wild-type mRNA immunological composition group (Wt), RSV F protein mutant mRNA immunological composition groups (F7, F7-3, F7-1 and F7-2) and F-protein immunological composition group were significantly higher than those in the PBS group (P<0.01).

### (4) Determination of IL-4 and IL-10 in spleen T lymphocyte culture supernatant by ELISA

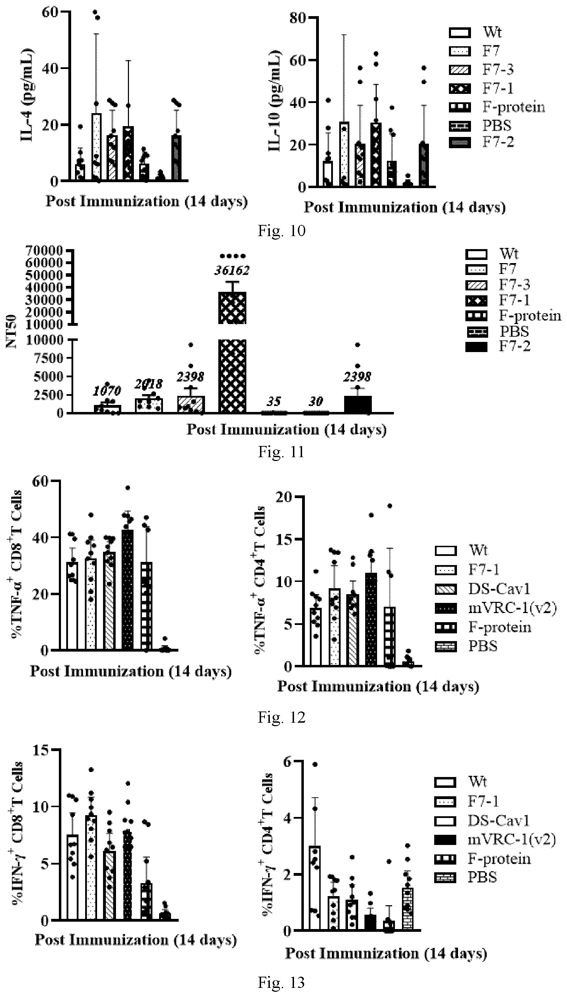

14 days after immunization, the expression of IL-4 and IL-10 in the lymphocyte supernatant was determined by ELISA. The results are shown in Figure 10.

The results showed that IL-4 in the F7-3 immunological composition group and F7-2 immunological composition group was significantly higher than that in the F-protein immunological composition group (P<0.01). IL-10 in the F7-1 immunological composition group was significantly higher than that in the F-protein immunological composition group (P<0.01). IL-4 and IL-10 in the RSV F protein wild-type mRNA immunological composition group (Wt), RSV F protein mutant mRNA immunological composition groups (F7, F7-3, F7-1 and F7-2) and F-protein immunological composition group were significantly higher than those in the PBS group (P<0.01).

The above results showed that RSV F protein wild-type mRNA immunological composition group (Wt), RSV F protein mutant mRNA immunological composition groups (F7, F7-3, F7-1 and F7-2), and F-protein immunological composition group can significantly stimulate the Th2 type cellular immune response in mice.

### (5) RSV neutralizing antibody titer in serum

14 days after the second immunization, the RSV neutralizing antibody titer in the mouse serum was measured by the plaque reduction assay directed to the RSV live virus. As shown in Figure 11, compared with RSV F protein wild-type mRNA immunological composition group (Wt), RSV F protein mutant mRNA immunological composition groups (F7-3, F7 and F7-2) and F-protein immunological composition group, the geometric mean titer of RSV neutralizing antibody in the F7-1 immunological composition group was 36162, which was significantly higher than other mRNA immunological composition groups (P<0.01).

In this Example, regarding the RSV F protein mutant mRNA immunological composition group wherein S155C/S190F/V207L/S290C substitutions and D486C/D489C substitutions were introduced and P27 sequence was replaced with the GS sequence as set forth in SEQ ID NO. 74 on the basis of the wild type RSV F protein; when the TM sequence was retained and the multimerization element was added (such as sample F7-1), the RSV neutralizing antibody titer in the mouse serum was significantly higher than that of only retaining the TM sequence (such as sample F7-2) or only adding multimerization element (such as sample F7).

### Example 7

Lipid nanoparticles containing RNAs encoding respiratory syncytial virus antigens, i.e., samples Wt, F7-1, DS-Cav1 and mVRC-1 (v2), were prepared according to the method described in Example 2, respectively.

The sequence of the RNA open reading frame in the lipid nanoparticle containing RNA encoding the respiratory syncytial virus antigen according to sample Wt is set forth in SEQ ID NO: 25, and the encoded respiratory syncytial virus antigen is RSV F protein wild type (the amino acid sequence is set forth in SEQ ID NO. 24).

The sequence of the RNA open reading frame in the lipid nanoparticle containing RNA encoding the respiratory syncytial virus antigen according to sample F7-1 is set forth in SEQ ID NO. 63, and the encoded respiratory syncytial virus antigen is a RSV F protein mutant (the amino acid sequence is set forth in SEQ ID NO. 62). The encoded amino acid sequence of the RSV F protein variant according to sample F7-1 is based on the RSV F protein wild type, with S155C/S190F/V207L/S290C/D486C/D489C substitutions, wherein the P27 sequence replaced by the GS sequence as set forth in SEQ ID NO. 74, and Fibritin Dominain (the amino acid sequence is set forth in SEQ ID NO. 7) added to the C-terminus.

The sequence of the RNA open reading frame in the lipid nanoparticle containing RNA encoding the respiratory syncytial virus antigen according to sample DS-Cav1 is set forth in SEQ ID NO. 27, and the encoded respiratory syncytial virus antigen is a RSV F protein mutant (the amino acid sequence is set forth in SEQ ID NO. 26). The encoded amino acid sequence of the RSV F protein variant according to sample DS-Cav1 is based on the RSV F protein wild type, with S155C/S190F/V207L/S290C substitutions.

The sequence of the RNA open reading frame in the lipid nanoparticle containing RNA encoding the respiratory syncytial virus antigen according to sample mVRC-1 (v2) is set forth in SEQ ID NO. 31, and the encoded respiratory syncytial virus antigen is a RSV F protein mutant (amino acid sequence is set forth in SEQ ID NO. 30).

The mRNA in the RNA immunological composition encoding respiratory syncytial virus antigen comprises, in addition to the above-mentioned reading frame sequence, a 5' cap (m7Gppp (5')), a 5' UTR (as set forth in SEQ ID NO. 16), a 3' UTR (as set forth in SEQ ID NO. 19) and a 3' polyA tail of 100 adenine nucleotides; and the uracil of the mRNA is replaced with a 5' pseudouracil.

In addition to the above lipid nanoparticles containing RNAs encoding respiratory syncytial virus antigens, an F protein (F-Protein) experimental group and a PBS blank control group were introduced into the experiment.

The vaccine immunogenicity of the prepared lipid nanoparticles of RNAs encoding respiratory syncytial virus antigens was evaluated according to the method of Example 4.

### (1) Detection of TNFα+ CD4+ and TNF-α+ CD8+ T cells by flow cytometry

14 days after immunization, mouse spleen lymphocytes were isolated, and the expression of TNF-α in CD3+/CD4+ and CD3+/CD8+ T lymphocytes was detected by flow cytometry. The results are shown in Figure 12.

The results showed that there was no significant difference in TNF-α+ CD8+ between the mRNA immunological composition groups and the F-protein immunological composition group (P>0.01). There was no significant difference in TNF-α+ CD4+ between the mRNA immunological composition groups and the F-protein immunological composition group (P>0.01). Compared with the PBS group, both the mRNA immunological composition groups and the F-protein immunological composition group could significantly stimulate Th1 type cellular immune response.

### (2) Detection of IFN-γ+ CD4+ and IFN-γ+ CD8+ T cells by flow cytometry

14 days after immunization, mouse spleen lymphocytes were isolated, and the expression of IFN-γ+ in CD3+/CD4+ and CD3+/CD8+ T lymphocytes was detected by flow cytometry. The results are shown in Figure 13.

The results showed that IFN-γ+CD8+ in the mRNA immunological composition group was significantly higher than that in the F-protein immunological composition group (P<0.01). Except for the mVRC-1 (v2) immunological composition group, IFN-γ+CD4+ in the other mRNA immunological composition groups was significantly higher than that in the F-protein immunological composition group (P<0.01). Compared with the PBS group, both the mRNA immunological composition groups and the F-protein immunological composition group could significantly stimulate Th1 type cellular immune response.

### (3) Determination of IFN-γ and IL-2 in culture supernatant from spleen T lymphocytes by ELISA

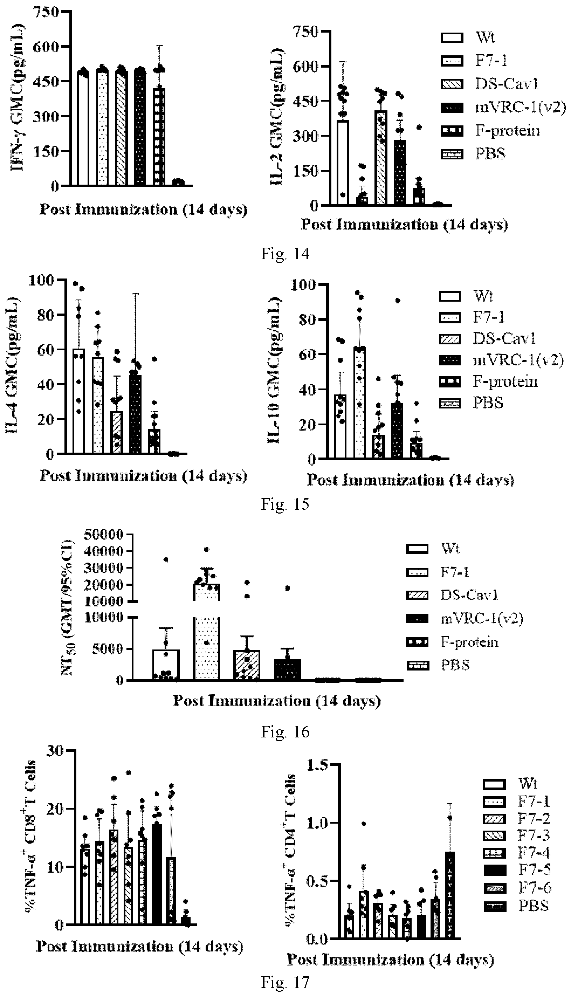

14 days after immunization, the expression of IFN-γ and IL-2 in the lymphocyte supernatant was determined by ELISA. The results are shown in Figure 14. The results showed that there was no significant difference in IFN-γ between the mRNA immunological composition groups and the F-protein immunological composition group (P>0.01). Except for the Wt immunological composition group, the IL-2 of the other mRNA immunological composition groups was significantly higher than that of the F-protein immunological composition group (P<0.01). Compared with the PBS group, both the mRNA immunological composition groups and the F-protein immunological composition group could significantly stimulate Th1 type cellular immune response.

### (4) Determination of IL-4 and IL-10 in culture supernatant from spleen T lymphocytes by ELISA

14 days after immunization, the expression of IL-4 and IL-10 in the lymphocyte supernatant was determined by ELISA. The results are shown in Figure 15. The results showed that: except for the DS-Cav1 immunological composition group, IL-4 of the other mRNA immunological composition groups was significantly higher than that of the F-protein immunological composition group (P<0.01). Except for the DS-Cav1 immunological composition group, IL-10 in the other mRNA immunological composition groups was significantly higher than that in the F-protein immunological composition group (P<0.01). Compared with the PBS group, both the mRNA immunological composition groups and the F-protein immunological composition group could significantly stimulate Th2-type cellular immune response.

### (5) RSV neutralizing antibody titer in serum

14 days after the second immunization, the RSV neutralizing antibody titer in the mouse serum was measured by the plaque reduction assay directed to the RSV live virus. As shown in Figure 16, compared with other mRNA immunological composition groups, the geometric mean titer of RSV neutralizing antibody in the F7-1 immunological composition group was 20821, which was significantly higher than other mRNA immunological composition groups (P<0.01).

### Example 8

Lipid nanoparticles containing RNAs encoding respiratory syncytial virus antigens, i.e., samples Wt, F7-1, F7-2, F7-3, F7-4, and F7-5 and F7-6, were prepared according to the method described in Example 2, respectively.

The sequence of the RNA open reading frame in the lipid nanoparticle containing RNA encoding the respiratory syncytial virus antigen according to sample Wt is set forth in SEQ ID NO. 25, and the encoded respiratory syncytial virus antigen is RSV F protein wild type (the amino acid sequence is set forth in SEQ ID NO. 24).

The sequence of the RNA open reading frame in the lipid nanoparticle containing RNA encoding the respiratory syncytial virus antigen according to sample F7-1 is set forth in SEQ ID NO. 63, and the encoded respiratory syncytial virus antigen is a RSV F protein mutant (the amino acid sequence is set forth in SEQ ID NO. 62). The encoded amino acid sequence of the RSV F protein variant according to sample F7-1 is based on the RSV F protein wild type, with S155C/S190F/V207L/S290C/D486C/D489C substitutions, wherein the P27 sequence is replaced by the GS sequence as set forth in SEQ ID NO. 74, and Fibritin Dominain (the amino acid sequence is set forth in SEQ ID NO. 7) is added to the C-terminus.

The sequence of the RNA open reading frame in the lipid nanoparticle containing RNA encoding the respiratory syncytial virus antigen according to sample F7-2 is set forth in SEQ ID NO. 65, and the encoded respiratory syncytial virus antigen is a RSV F protein mutant (amino acid sequence set forth in SEQ ID NO. 64). The encoded amino acid sequence of the RSV F protein variant according to sample F7-2 is based on the RSV F protein wild type, with S155C/S190F/V207L/S290C/D486C/D489C substitutions, wherein the P27 sequence is replaced by the GS sequence as set forth in SEQ ID NO. 74.

The sequence of the RNA open reading frame in the lipid nanoparticle containing RNA encoding the respiratory syncytial virus antigen according to sample F7-3 is set forth in SEQ ID NO. 67, and the encoded respiratory syncytial virus antigen is a RSV F protein mutant (the amino acid sequence is set forth in SEQ ID NO. 66). The encoded amino acid sequence of the RSV F protein variant according to sample F7-3 is based on the RSV F protein wild type, with S155C/S190F/V207L/S290C/D486C/D489C substitutions, wherein GCN4 Dominain (the amino acid sequence is set forth in SEQ ID NO. 12) is added to the C-terminus.

The sequence of the RNA open reading frame in the lipid nanoparticle containing RNA encoding the respiratory syncytial virus antigen according to sample F7-4 is set forth in SEQ ID NO. 69, and the encoded respiratory syncytial virus antigen is a RSV F protein mutant (the amino acid sequence is set forth in SEQ ID NO. 68). The encoded amino acid sequence of the RSV F protein variant according to sample F7-4 is based on the RSV F protein wild type, with S155C/S190F/V207L/S290C substitutions, wherein the P27 sequence is replaced by the GS sequence as set forth in SEQ ID NO. 74, and Fibritin Dominain (the amino acid sequence is set forth in SEQ ID NO. 7) isa added to the C-terminus.

The sequence of the RNA open reading frame in the lipid nanoparticle containing RNA encoding the respiratory syncytial virus antigen according to sample F7-5 is set forth in SEQ ID NO. 71, and the encoded respiratory syncytial virus antigen is a RSV F protein mutant (the amino acid sequence is set forth in SEQ ID NO. 70). The encoded amino acid sequence of the RSV F protein variant according to sample F7-5 is based on the RSV F protein wild type, with S155C/S190F/V207L/S290C substitutions, wherein Fibritin Dominain (the amino acid sequence is set forth in SEQ ID NO. 7) is added to the C-terminus.

The sequence of the RNA open reading frame in the lipid nanoparticle containing RNA encoding the respiratory syncytial virus antigen according to sample F7-6 is set forth in SEQ ID NO. 73, and the encoded respiratory syncytial virus antigen is a RSV F protein mutant (the amino acid sequence is set forth in SEQ ID NO. 72). The encoded amino acid sequence of the RSV F protein variant according to sample F7-6 is based on the RSV F protein wild type, with S155C/S190F/V207L/S290C/D486C/D489C substitutions, wherein Fibritin Dominain (the amino acid sequence is set forth in SEQ ID NO. 7) is added to the C-terminus.

The mRNA in the RNA immunological composition encoding respiratory syncytial virus antigen comprises, in addition to the above-mentioned reading frame sequence, a 5' cap (m7Gppp (5')), a 5' UTR (as set forth in SEQ ID NO. 16), a 3' UTR (as set forth in SEQ ID NO. 19) and a 3' polyA tail of 100 adenine nucleotides; and the uracil of the mRNA is replaced with a 5' pseudouracil.

In addition to the above lipid nanoparticles containing RNAs encoding respiratory syncytial virus antigens, a PBS blank control group was introduced into the experiment.

The vaccine immunogenicity of the prepared lipid nanoparticles of RNAs encoding respiratory syncytial virus antigens was evaluated according to the method of Example 4.

### (1) Detection of TNFα+ CD4+ and TNF-α+ CD8+ T cells by flow cytometry

14 days after immunization, mouse spleen lymphocytes were isolated, and the expression of TNF-α in CD3+/CD4+ and CD3+/CD8+ T lymphocytes was detected by flow cytometry. The results are shown in Figure 17.

The results showed that there was no significant difference in TNF-α+ CD8+ in the F7-1 immunological composition group compared with other mRNA immunological composition groups (P>0.01). No significant increase in TNF-α+ CD4+ was detected in the mRNA immunological composition groups (P>0.01). Compared with the PBS group, the mRNA immunological composition groups could significantly stimulate Th1 type cellular immune response.

### (2) Detection of IFN-γ+ CD4+ and IFN-γ+ CD8+ T cells by flow cytometry

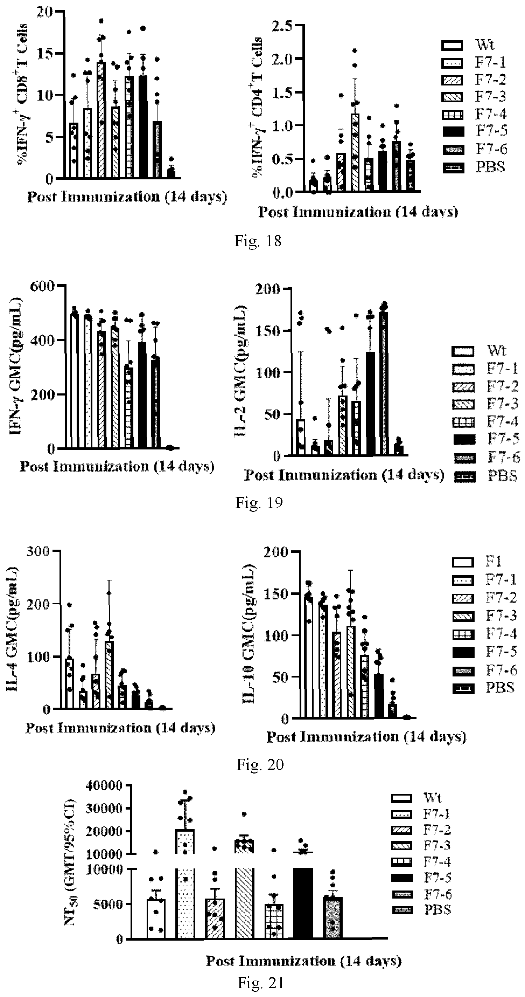

14 days after immunization, mouse spleen lymphocytes were isolated, and the expression of IFN-γ in CD3+/CD4+ and CD3+/CD8+ T lymphocytes was detected by flow cytometry. The results are shown in Figure 18.

The results showed that: except for the F7-2 immunological composition group, there was no significant difference in IFN-γ+CD8+ between the F7-1 immunological composition group and other mRNA immunological composition groups. No significant increase in IFN-γ+CD4+ was detected in the mRNA immunological composition group (P>0.01). Compared with the PBS group, the mRNA immunological composition groups could significantly stimulate Th1 type cellular immune response.

### (3) Determination of IFN-γ and IL-2 in culture supernatant from spleen T lymphocytes by ELISA

14 days after immunization, the expression of IFN-γ and IL-2 in the lymphocyte supernatant was determined by ELISA. The results are shown in Figure 19. The results showed that: except for the F7-4 and F7-6 immunological composition groups, there was no significant difference in IFN-γ between the F7-1 immunological composition group and other mRNA immunological composition groups. IL-2 detected in the F7-1 immunological composition group was significantly higher than that in other mRNA immunological composition groups (P<0.01). Compared with the PBS group, the mRNA immunological composition groups could significantly stimulate Th1 type cellular immune response.

### (4) Determination of IL-4 and IL-10 in culture supernatant from spleen T lymphocytes by ELISA

14 days after immunization, the expression of IL-4 and IL-10 in the lymphocyte supernatant was determined by ELISA. The results are shown in Figure 20. The results showed that: except for Wt and F7-3 immunological composition groups, there was no significant difference in IL-4 between the F7-1 immunological composition group and other mRNA immunological composition groups. Except for the Wt, F7-2 and F7-3 immunological composition groups, IL-10 in the F7-1 immunological composition group was significantly higher than that in other mRNA immunological composition groups. Compared with the PBS group, the mRNA immunological composition groups could significantly stimulate Th2 type cellular immune response.

### (5) RSV neutralizing antibody titer in serum

14 days after the second immunization, the RSV neutralizing antibody titer in the mouse serum was measured by the plaque reduction assay directed to the RSV live virus. The results are shown in Figure 21. As shown in the figure, compared with the mRNA immunological composition groups, the geometric mean titer of RSV neutralizing antibody in the F7-1 immunological composition group was 20730, which was significantly higher than other mRNA immunological composition groups and the F-Protein immunological composition group (P<0.01). The geometric mean titer of RSV neutralizing antibody in the F7-3 immunological composition group was 16026, and the geometric mean titer of RSV neutralizing antibody in the F7-5 immunological composition group was 10871, which were significantly higher than that in Wt, F7-2, F7-4, F7-6 immunological composition groups and F-Protein immunological composition group (P<0.01).

### Example 9

Lipid nanoparticles containing RNAs encoding respiratory syncytial virus antigens, namely samples Wt, F7-1, F7-a and F7-b, were prepared according to the method described in Example 2, respectively.

The sequence of the RNA open reading frame in the lipid nanoparticle containing RNA encoding the respiratory syncytial virus antigen according to sample Wt is set forth in SEQ ID NO. 25, and the encoded respiratory syncytial virus antigen is the RSV F protein wild type (the amino acid sequence is set forth in SEQ ID NO. 24).

The sequence of the RNA open reading frame in the lipid nanoparticle containing RNA encoding the respiratory syncytial virus antigen according to sample F7-1 is set forth in SEQ ID NO. 63, and the encoded respiratory syncytial virus antigen is a RSV F protein mutant (the amino acid sequence is set forth in SEQ ID NO. 62).

The sequence of the RNA open reading frame in the lipid nanoparticle containing RNA encoding the respiratory syncytial virus antigen according to sample F7-a is set forth in SEQ ID NO. 77, and the encoded respiratory syncytial virus antigen is a RSV F protein mutant (the amino acid sequence is set forth in SEQ ID NO. 76).

The sequence of the RNA open reading frame in the lipid nanoparticle containing RNA encoding the respiratory syncytial virus antigen according to sample F7-b is set forth in SEQ ID NO. 79, and the encoded respiratory syncytial virus antigen is a RSV F protein mutant (the amino acid sequence is set forth in SEQ ID NO. 78).

In addition to the above-mentioned nucleotide ORF sequences, the sequences of these RNA vaccines also comprise a 5' cap (m7Gppp (5')), a 5' UTR (as set forth in SEQ ID NO. 16), a 3' UTR (as set forth in SEQ ID NO. 19) and a 3' polyA tail of 100 adenine nucleotides; and the uracil of the mRNA is replaced with a 5' pseudouracil.

In addition to the above lipid nanoparticles containing RNAs encoding respiratory syncytial virus antigens, a PBS blank control group was introduced into the experiment.

The prepared lipid nanoparticles encoding RNAs encoding respiratory syncytial virus antigens were evaluated for RSV neutralizing antibody titer in serum according to the method of Example 4.

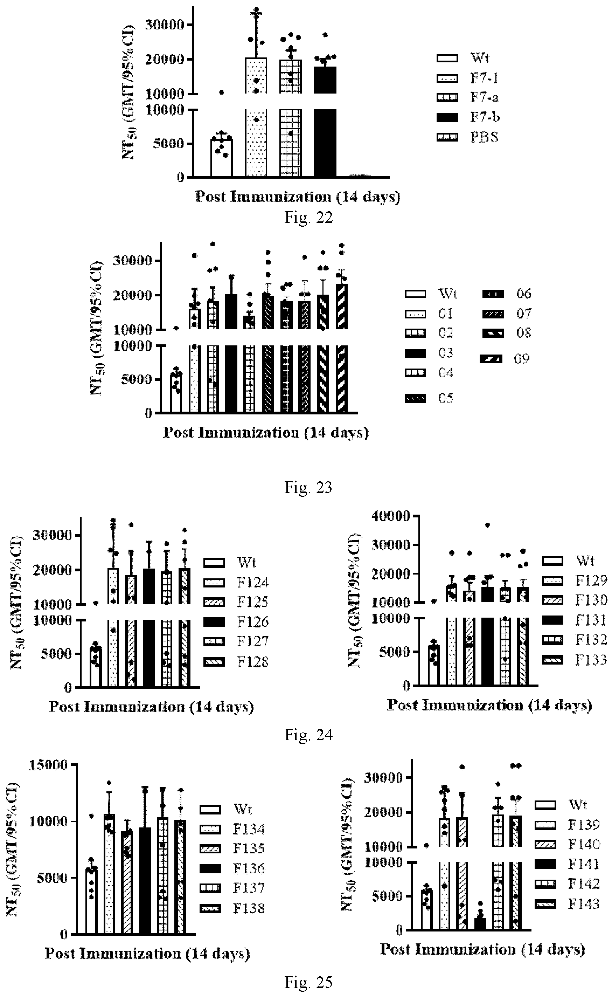

14 days after the second immunization, the RSV neutralizing antibody titer in the mouse serum was measured by the plaque reduction assay directed to the RSV live virus. As shown in Figure 22, compared with the Wt immunological composition group, the geometric mean titer of the RSV neutralizing antibody in the F7-1 immunological composition group, F7-a immunological composition group, and F7-b immunological composition group all exceeded 18,000, significantly higher than the Wt immunological composition group (P<0.01).

### Example 10

The nucleotide sequences described in Table 4 below are mRNA sequences. The cells were transfected with the mRNA shown in Table 4 below, and the expression of RSV F full-length protein in the cells was detected. The results are shown in Table 4 below.

The details of the method are as follows: HEK293 cells were lysed 24 hours after transfection with each mRNA, and the protein of interest was specifically detected by SDS-PAGE immunoblotting using 10 µg total protein as the loading amount. In this Example, anti-RSV F protein antibody was used as the primary antibody and goat anti-mouse-HRP antibody was used as the secondary antibody for incubation, respectively, and then chromogenic reaction was performed. Regarding analysis into the detection results of protein expression, internal reference β-actin was used for standardized quantification, while the group with cells that were not transfected with mRNA was set as negative control; and the differences in the amount of protein expressed by cells transfected with different mRNAs were compared. The test results showed that the expression of the full length RSV F protein by the different mRNAs can be detected. The expression of each sequence was measured by relative OD value, as shown in Table 4 below, wherein the relative OD value was calculated as follows: OD value of sample n/OD value of sample 1.

**Table 4**

| Serial number | ORF nucleotide sequence | 3'UTR | 5'UTR | Relative expression |
|---|---|---|---|---|
| 1 | SEQ ID NO. 63 | SEQ ID NO. 21 | SEQ ID NO. 18 | 1 |
| 2 | SEQ ID NO. 63 | SEQ ID NO. 20 | SEQ ID NO. 18 | 1.1 |
| 3 | SEQ ID NO. 63 | SEQ ID NO. 19 | SEQ ID NO. 18 | 1.2 |
| 4 | SEQ ID NO. 63 | SEQ ID NO. 21 | SEQ ID NO. 17 | 0.9 |
| 5 | SEQ ID NO. 63 | SEQ ID NO. 20 | SEQ ID NO. 17 | 1.2 |
| 6 | SEQ ID NO. 63 | SEQ ID NO. 19 | SEQ ID NO. 17 | 1.1 |
| 7 | SEQ ID NO. 63 | SEQ ID NO. 21 | SEQ ID NO. 16 | 1.1 |
| 8 | SEQ ID NO. 63 | SEQ ID NO. 20 | SEQ ID NO. 16 | 1.2 |
| 9 | SEQ ID NO. 63 | SEQ ID NO. 19 | SEQ ID NO. 16 | 1.4 |

### Example 11

The individual RNA sequences 1 to 9 in Table 4 of Example 10 were prepared into lipid nanoparticles containing RNAs encoding respiratory syncytial virus antigens according to the method described in Example 2, which were designated as samples 01 to 09 respectively.

In addition to the above-mentioned nucleotide sequences, the sequences of these RNA vaccines also comprise a 5' cap (m7Gppp (5')) and a 3' polyA tail of 100 adenine nucleotides; and the uracil of the mRNA is replaced with a 5' pseudouracil.

In addition to the above lipid nanoparticles containing RNAs encoding respiratory syncytial virus antigens, a PBS blank control group was introduced into the experiment. Sample Wt was prepared according to Example 9.

The prepared lipid nanoparticles encoding RNAs encoding respiratory syncytial virus antigens were evaluated for RSV neutralizing antibody titer in serum according to the method of Example 4.

14 days after the second immunization, the RSV neutralizing antibody titer in the mouse serum was measured by the plaque reduction assay directed to the RSV live virus. As shown in Figure 23, compared with the Wt immunological composition group, the geometric mean titer of the RSV neutralizing antibody in samples 01 to 09 all exceeded 10,000, which was significantly higher than that of the Wt immunological composition group (P<0.01); and the geometric mean titer of the RSV neutralizing antibody in sample 09 was slightly higher than that of other samples 01 to 08.

### Example 12

Lipid nanoparticles containing RNAs encoding respiratory syncytial virus antigens were prepared according to the method described in Example 2, which were designated as samples Wt and F124 to F138, respectively.

The sequence of the RNA open reading frame in the lipid nanoparticle containing RNA encoding the respiratory syncytial virus antigen according to sample Wt is set forth in SEQ ID NO. 25, and the encoded respiratory syncytial virus antigen is the wild type RSV F protein (the amino acid sequence is set forth in SEQ ID NO. 24).

The sequence of the RNA open reading frame in the lipid nanoparticle containing RNA encoding the respiratory syncytial virus antigen according to samples 124 to 128 are set forth in SEQ ID NO. 63 and SEQ ID NOs. 84 to 87, respectively, and the encoded respiratory syncytial virus antigen is a RSV F protein mutant (the amino acid sequence is set forth in SEQ ID NO. 62).

The sequence of the RNA open reading frame in the lipid nanoparticle containing RNA encoding the respiratory syncytial virus antigen according to samples 129 to 133 are set forth in SEQ ID NO. 67 and SEQ ID NOs. 88 to 91, respectively, and the encoded respiratory syncytial virus antigen is a RSV F protein mutant (the amino acid sequence is set forth in SEQ ID NO. 66).

The sequence of the RNA open reading frame in the lipid nanoparticle containing RNA encoding the respiratory syncytial virus antigen according to samples 134 to 138 are set forth in SEQ ID NO. 71 and SEQ ID NOs. 92 to 95, respectively, and the encoded respiratory syncytial virus antigen is a RSV F protein mutant (the amino acid sequence is set forth in SEQ ID NO. 70).

The sequence of the RNA open reading frame in the lipid nanoparticle containing RNA encoding the respiratory syncytial virus antigen according to samples 139 to 143 are set forth in SEQ ID NO. 77 and SEQ ID NOs. 96 to 99, respectively, and the encoded respiratory syncytial virus antigen is a RSV F protein mutant (the amino acid sequence is set forth in SEQ ID NO. 76).

The sequence of the RNA open reading frame in the lipid nanoparticle containing RNA encoding the respiratory syncytial virus antigen according to samples 144 to 148 are set forth in SEQ ID NO. 79 and SEQ ID NOs. 100 to 103, respectively, and the encoded respiratory syncytial virus antigen is a RSV F protein mutant (the amino acid sequence is set forth in SEQ ID NO. 78).

The sequence of the RNA open reading frame in the lipid nanoparticle containing RNA encoding the respiratory syncytial virus antigen is set forth in SEQ ID NO. 103 and the encoded respiratory syncytial virus antigen is a RSV F protein mutant (the amino acid sequence is set forth in SEQ ID NO. 78).

In addition to the above-mentioned nucleotide ORF sequences, the sequences of these RNA vaccines also comprise a 5' cap (m7Gppp (5')), a 5' UTR (as set forth in SEQ ID NO. 16), a 3' UTR (as set forth in SEQ ID NO. 19) and a 3' polyA tail of 100 adenine nucleotides; and the uracil of the mRNA is replaced with a 5' pseudouracil.

In addition to the above lipid nanoparticles containing RNAs encoding respiratory syncytial virus antigens, a PBS blank control group was introduced into the experiment.

The prepared lipid nanoparticles encoding RNAs encoding respiratory syncytial virus antigens were evaluated for RSV neutralizing antibody titer in serum according to the method of Example 4.

14 days after the second immunization, the RSV neutralizing antibody titer in the mouse serum was measured by the plaque reduction assay directed to the RSV live virus. As shown in Figures 24 to 26, compared with the Wt immunological composition group, the geometric mean titer of the RSV neutralizing antibody in the F124 to F148 immunological composition groups all exceeded 10,000, which was significantly higher than that of the Wt immunological composition group (P<0.01).

The titer of the RSV neutralizing antibody in samples 124-128 and 140-148 wherein the respiratory syncytial virus antigen is RSV F protein mutant and has edited amino acid sequence, such as SEQ ID NO. 62, SEQ ID NO. 76 and SEQ ID NO. 78, was higher than the neutralizing antibody titer in other samples.

### Example 13

BALB/c mice (female, 5 weeks old, with an average body weight of about 19 g, purchased from Zhuhai Baishitong Biotechnology Co., Ltd.) were selected for vaccine immunogenicity evaluation. The experimental animals were randomly divided into 5 groups, with 6 mice in each group. The dosing regime was as follows: (1) Vehicle group: intramuscular injection of 50 µg/mouse PBS buffer containing empty lipid nanoparticles; (2) F protein group: intramuscular injection of 50 µg/mouse PBS buffer containing 0.05 µg pre-fusion hRSV F protein; (3) F7-1 (5 µg) group: intramuscular injection of 25 µg/mouse PBS buffer containing 5 µg of lipid nanoparticles containing RNA encoding the respiratory syncytial virus antigen, wherein the open reading frame sequence of the RNA is set forth in SEQ ID NO. 63; (4) F7-1 (15 µg) group: intramuscular injection of 75 µg/mouse PBS buffer containing 15 µg of lipid nanoparticles containing RNA encoding the respiratory syncytial virus antigen, wherein the open reading frame sequence of the RNA is set forth in SEQ ID NO. 63; (5) Health group: no dosing. The same dose of booster immunization was given on the 21^{st} day after vaccination, and serum samples were collected on the 35^{th} day after vaccination. On the 40^{th} day after vaccination, the mice were sacrifieced and lung tissues were collected. On the 35^{th} day after vaccination, mice in groups 1 to 4 were inoculated with RSV/A2 virus by nasal drops. The weight and weight changes of mice were monitored during the experiment; and RSV-F protein-specific IgG antibodies, neutralizing activity in serum, virus titer in the lung tissue of mice after RSV infection, and lung tissue pathology of mice after RSV infection were determined. The method for determining the RSV-F protein-specific IgG antibodies and neutralizing activity in serum is shown in Example 4 of the present disclosure. Lung tissue lesions (perivasculitis, peribronchiolitis, alveolitis, and interstitial pneumonia) were evaluated by HE staining. Virus titer in the lung tissue was detected by plaque assay.

Lipid nanoparticles containing RNA encoding the respiratory syncytial virus antigen were prepared according to the method described in Example 2, which was designated as sample F7-1. In addition to the above-mentioned nucleotide ORF sequence, the sequences of these RNA vaccines also comrpise a 5' cap (m7Gppp (5')), a 5' UTR (as set forth in SEQ ID NO. 16), a 3' UTR (as set forth in SEQ ID NO. 19) and a 3' polyA tail of 100 adenine nucleotides; and the uracil of the mRNA is replaced with a 5' pseudouracil.

### (1) Mouse weight and weight changes

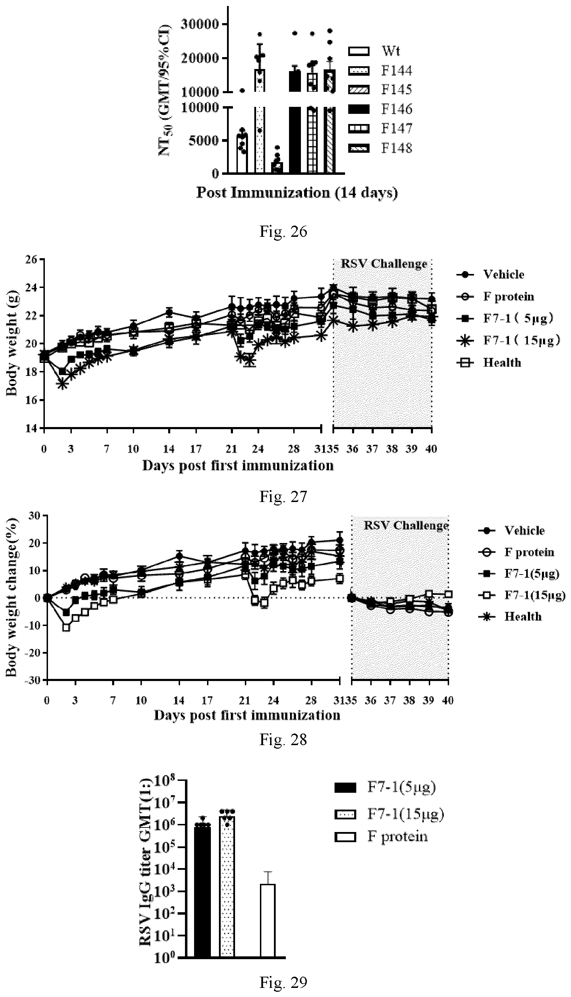

The body weight and body weight changes of the mice during the experiment are shown in Figures 27 and 28. On the 2^{nd} to 5^{th} day after the first vaccination, the body weight of the F7-1 (5 µg) and F7 -1 (15 µg) immunization groups decreased significantly (Figure 27). The weight of the F7 -1 (5 µg) immunization group decreased by about 5% on the 2^{nd} day after the first vaccination, and the weight of the F7-1 (15 µg) immunization group decreased by about 10% on the 2^{nd} day after the first vaccination (Figure 28). The body weight of the F7-1 (5µg) and F7-1 (15µg) immunization groups was significantly reduced (Figure 27) on the 1^{st} day after the second vaccination (22 days after the first vaccination). The weight of the F7-1 (5µg) immunization group decreased by about 5%; and the weight of the F7-1 (15 µg) immunization group decreased by about 10% on the 2^{nd} day after the second vaccination (Figure 28). Compared with the body weight prior to intranasal inoculation of RSV/A2 virus, the body weight of the vehicle immunization group decreased by about 3%, the F protein immunization group decreased by about 5%, and the F7-1 (5 µg) immunization group decreased by about 4%; while the body weight of the F7-1 (15µg) immunization group increased by 1%, on the 5^{th} day after challenge.

### (2) Determination of RSV-F protein-specific IgG in serum by ELISA

On the 35^{th} day after the first vaccination, RSV-F protein-specific IgG antibodies in mouse serum were detected by ELISA. The serum was diluted 100 times to 8 million times, and OD450 was measured by indirect ELISA.

When the sample OD450 is ≥ 2 times the average OD450 of the negative control group with minimum dilution factor (1:100), it will be considered positive (Bao-Zhong Wang, et al.. PLoS ONE. 2010; Teena Mohan, et al.. J Control Release. 2016). The final dilution titer was reached when the serum sample of the F7-1 (15µg) immunization group was diluted to 8 million times.

Statistics on RSV-F protein-specific IgG antibody titer in serum are shown in Figure 29. The results showed that the IgG titer GMT value in the F7-1 (15 µg) immunization group was the highest. The IgG antibody titer in the F7-1 (5 µg) and F7 -1 (15 µg) immunization groups were significantly higher than that of the F protein immunization group.

### (3) RSV neutralizing antibody titer in serum

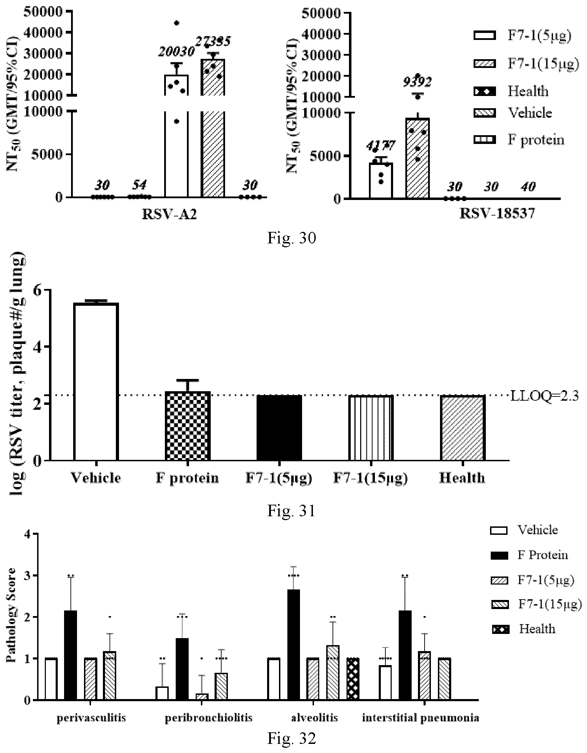

On the 35^{th} day after the first vaccination, the RSV neutralizing antibody titer in the mouse serum was measured by the plaque reduction assay directed to RSV-A/B strain viru. As shown in the left panel of Figure 30, with respect to the RSV-A2 strain, the neutralizing antibody titer of the F7-1 (5µg) (GMT=20030) and F7-1 (15µg) (GMT=27335) immunization groups were significantly higher than that of the F protein (GMT=54) immunization group (P<0.05), but there was no significant difference between the F7-1 (5 µg) immunization group and the F7-1 (15 µg) immunization group, indicating that there was no significant difference between the high and low dose groups of F7-1 vaccine. As shown in the right panel of Figure 30, with respect to the RSV-18537 strain, the neutralizing antibody titer of the F7-1 (5 µg) (GMT=4177) and F7-1 (15 µg) (GMT=9392) immunization groups was significantly higher than that of the F protein (GMT=37) immunization group (P<0.05). There was no significant difference in the neutralizing antibody titer between the F7-1 (5 µg) immunization group and the F7-1 (15 µg) immunization group (P>0.05), indicating that there was no significant difference between the F7-1 high and low dose groups.

### (4) Virus titer in the lung tissue of mice after RSV infection

5 days after RSV infection, the RSV virus titer in the right lung tissue homogenate of mice after intranasal inoculation of RSV/A2 virus was measured by the plaque reduction neutralization test (see Figure 31). The virus titer in the lung tissue of the Vehicle immunization group was significantly higher than that of the F protein, F7 -1 (5 µg), F7 -1 (15 µg) and Health groups. Among them, F7-1 (5 µg), F7-1 (15 µg) and Health group were all at the lower limit of quantitation (LLOQ). The results showed that F protein (RSV inactivated vaccine), F7-1 (5 µg) and F7-1 (15 µg) mRNA vaccines could significantly reduce the virus titer in the lung tissue of mice in the immunization group.

### (5) Lung histopathology of mice after RSV infection

5 days after RSV infection, lung tissue lesions (perivasculitis, peribronchiolitis, alveolitis, and interstitial pneumonia) were evaluated by HE staining. As shown in Figure 32, the perivasculitis, peribronchiolitis, alveolitis, and interstitial pneumonia in the F protein immunization group were significantly higher than those in the Vehicle immunization group. Vehicle, 007-5µg and 007-15µg immunization groups all showed mild pathological reactions after RSV infection. Taken together, these results indicate that F7-1 (5µg) and F7-1 (15µg) immunization groups can induce higher titers of IgG-specific antibodies and neutralizing antibodies, protecting animals from RSV infection without causing vaccine-enhanced respiratory disease (VERD).

Finally, it should be noted that the above embodiments are only intended to be illustrative of the technical solutions of the present disclosure, instead of limiting; although the present disclosure has been described in detail with reference to the foregoing embodiments, those of ordinary skill in the art should appreciate that, it is still possible to modify the technical solutions recited in the foregoing embodiments, or to equivalently replace some or all of the technical features; and these modifications or substitutions do not render the essence of the corresponding technical solutions deviating from the scope of the technical solutions of various embodiments of the present disclosure.

## Claims

1. An immunological composition, comprising or encoding a human respiratory syncytial virus antigen, wherein the immunological composition is selected from a nucleic acid immunological composition, a polypeptide immunological composition or a viral immunological composition;
preferably, the antigen is a pre-fusion hRSV F protein, and the amino acid sequence of the pre-fusion hRSV F protein comprises a TM sequence and a multimerization element; preferably, the amino acid sequence of the multimerization element is set forth in SEQ ID NOs. 1-12 or SEQ ID NOs. 14-15; preferably, the amino acid sequence of the multimerization element is set forth in one of SEQ ID NO. 7 and SEQ ID NO. 12.

2. The immunological composition of claim 1, wherein the pre-fusion hRSV F protein has the P27 sequence replaced with a GS sequence; preferably, the GS sequence comprises (GnS)m, (GGGGS)o, GGSGGGGSGG, GGSGGGGG, GSGSGSGS, (Gly)p, (EAAAK)q, SEQ ID NOs. 48-61, or SEQ ID NO. 74 (wherein n is 1, 2, 3, 4, 5, 6, 7, 8, 9, 10, 15 or 20; m is 1, 2, 3, 4, 5, 6, 7, 8, 9, 10, 15 or 20; o is an integer from 1 to 5; p is an integer from 1 to 40; q is an integer from 1 to 5); preferably, the amino acid sequence of the GS sequence is set forth in SEQ ID NO. 74;
preferably, the pre-fusion hRSV F protein comprises at least one of an S46G substitution, an N67I substitution, an E92D substitution, a P102A substitution, an A149C substitution, an S155C substitution, an S190F substitution, a V207L substitution, an S290C substitution, an L373R substitution, an S215P substitution, an I379V substitution, an M447V substitution, a Y458C substitution, a K465Q substitution, a D486C substitution, and a D489C substitution.

3. The immunological composition of claim 1 or 2, wherein the amino acid sequence of the pre-fusion hRSV F protein comprises at least one of an S155C substitution, an S190F substitution, a V207L substitution, an S290C substitution, a D486C substitution, and a D489C substitution;
preferably, the amino acid sequence of the pre-fusion hRSV F protein comprises at least one of an S155C substitution, an S190F substitution, a V207L substitution, an S290C substitution, and/or the pre-fusion hRSV F protein has the P27 sequence replaced with a GS sequence;
preferably, the amino acid sequence of the pre-fusion hRSV F protein comprises an S155C substitution, an S190F substitution, a V207L substitution, and an S290C substitution; the pre-fusion hRSV F protein has the P27 sequence replaced with the GS sequence as set forth in SEQ ID NO. 74; the pre-fusion hRSV F protein comprises a multimerization element as set forth in one of SEQ ID NO. 7 and SEQ ID NO. 12;
preferably, the amino acid sequence of the pre-fusion hRSV F protein comprises an S155C substitution, an S190F substitution, a V207L substitution, and an S290C substitution; the pre-fusion hRSV F protein has the P27 sequence replaced with the GS sequence as set forth in SEQ ID NO. 74; the pre-fusion hRSV F protein comprises a multimerization element as set forth in one of SEQ ID NO. 7 and SEQ ID NO. 12; the amino acid sequence of the pre-fusion hRSV F protein further comprises at least one of an S46G substitution, an N67I substitution, an E92D substitution, a P102A substitution, an A149C substitution, an L373R substitution, an S215P substitution, an I379V substitution, an M447V substitution, a Y458C substitution, a K465Q substitution, a D486C substitution, and a D489C substitution;
preferably, the amino acid sequence of the pre-fusion hRSV F protein comprises at least one of an S155C substitution, an S190F substitution, a V207L substitution, an S290C substitution, a D486C substitution, and a D489C substitution, and/or the pre-fusion hRSV F protein has the P27 sequence replaced with a GS sequence;
preferably, the amino acid sequence of the pre-fusion hRSV F protein comprises an S155C substitution, an S190F substitution, a V207L substitution, an S290C substitution, a D486C substitution, and a D489C substitution, the pre-fusion hRSV F protein has the P27 sequence replaced with the GS sequence as set forth in SEQ ID NO. 74, and the pre-fusion hRSV F protein comprises a multimerization element as set forth in one of SEQ ID NO. 7 and SEQ ID NO. 12;
preferably, the amino acid sequence of the pre-fusion hRSV F protein comprises an S155C substitution, an S190F substitution, a V207L substitution, an S290C substitution, a D486C substitution, and a D489C substitution, the pre-fusion hRSV F protein has the P27 sequence replaced with the GS sequence as set forth in SEQ ID NO. 74, and the pre-fusion hRSV F protein comprises a multimerization element as set forth in one of SEQ ID NO. 7 and SEQ ID NO. 12; the amino acid sequence of the pre-fusion hRSV F protein further comprises at least one of an S46G substitution, an N67I substitution, an E92D substitution, a P102A substitution, an A149C substitution, an L373R substitution, an S215P substitution, an I379V substitution, an M447V substitution, a Y458C substitution, and a K465Q substitution.

4. The immunological composition of claim 1 or 2, wherein the pre-fusion hRSV F protein comprises an amino acid sequence in which at least one of the amino acids in the F2 sequence, the RR1 sequence, the RR2 sequence, the 525-529 sequence or the 551-574 sequence is deleted sequentially or intermittently.

5. The immunological composition of claim 1 or 2, wherein the pre-fusion hRSV F protein has an amino acid sequence as set forth in SEQ ID NO. 62, SEQ ID NO. 64, SEQ ID NO. 66, SEQ ID NO. 68, SEQ ID NO. 70, SEQ ID NO. 72, SEQ ID NO. 76, or SEQ ID NO. 78;
preferably, the pre-fusion hRSV F protein has an amino acid sequence as set forth in SEQ ID NO. 62, SEQ ID NO. 64, SEQ ID NO. 66, SEQ ID NO. 70, SEQ ID NO. 76, or SEQ ID NO. 78;
preferably, the pre-fusion hRSV F protein has an amino acid sequence as set forth in SEQ ID NO. 62, SEQ ID NO. 66, SEQ ID NO. 70, SEQ ID NO. 76, or SEQ ID NO. 78.

6. The immunological composition of claim 5, wherein the multimerization element of the pre-fusion hRSV F protein can further be selected from the amino acid sequences as set forth in SEQ ID NOs. 1-12 and SEQ ID NOs. 14-15.

7. The immunological composition of claim 1 or 2, wherein the immunological composition is a nucleic acid immunological composition; the nucleic acid immunological composition comprises a nucleic acid molecule; the nucleic acid molecule comprises a DNA molecule and/or an RNA molecule; preferably, the DNA molecule comprises a stranded DNA molecule and/or a circular DNA molecule; preferably, the RNA molecule comprises an mRNA or a circular RNA; preferably, the mRNA molecule comprises an open reading frame (ORF), and the open reading frame (ORF) has a nucleotide sequence as set forth in SEQ ID NO. 63, SEQ ID NO. 65, SEQ ID NO. 67, SEQ ID NO. 69, SEQ ID NO. 71, SEQ ID NO. 73, SEQ ID NO. 77, SEQ ID NO. 79, or SEQ ID NOs. 84 to 103;
preferably, the mRNA molecule comprises an open reading frame (ORF), and the open reading frame (ORF) has a nucleotide sequence as set forth in SEQ ID NO. 63, SEQ ID NO. 67, SEQ ID NO. 71, SEQ ID NO. 77, SEQ ID NO. 79, or SEQ ID NOs. 84 to 103;
preferably, the mRNA molecule comprises an open reading frame (ORF), and the open reading frame (ORF) has a nucleotide sequence as set forth in SEQ ID NO. 63, SEQ ID NO. 71, SEQ ID NO. 77, SEQ ID NO. 79, SEQ ID NOs. 84-87, or SEQ ID NOs. 92-103;
preferably, the mRNA molecule comprises an open reading frame (ORF), and the open reading frame (ORF) has a nucleotide sequence as set forth in SEQ ID NO. 63, SEQ ID NO. 77, SEQ ID NO. 79, SEQ ID NOs. 84-87, or SEQ ID NOs. 96-103;
preferably, the mRNA molecule comprises an open reading frame (ORF), and the open reading frame (ORF) has a nucleotide sequence as set forth in SEQ ID NO. 67;
preferably, the mRNA molecule comprises an open reading frame (ORF), and the open reading frame (ORF) has a nucleotide sequence as set forth in SEQ ID NO. 71;
preferably, the nucleotide sequence of the multimerization element in the open reading frame (ORF) can also be set forth in SEQ ID NOs. 36-47, or SEQ ID NO. 13.

8. The immunological composition of claim 1 or 2, wherein the immunological composition comprises a delivery formulation; preferably, the delivery formulation comprises lipid nanoparticles or cationic liposomes.

9. A method for preparing an immunological composition of claim 7, wherein the mRNA and a delivery formulation are mixed to form the immunological composition; the delivery formulation comprises lipid nanoparticles or cationic liposomes.

10. An isolated mRNA, wherein the nucleic acid sequence of the coding region of the mRNA encodes a pre-fusion hRSV F protein, and the amino acid sequence of the pre-fusion hRSV F protein comprises a TM sequence and a multimerization element; preferably, the amino acid sequence of the multimerization element is set forth in SEQ ID NOs. 1-12, or SEQ ID NOs. 14-15; preferably, the amino acid sequence of the multimerization element is set forth in one of SEQ ID NO. 7 and SEQ ID NO. 12;
preferably, the mRNA further comprises: at least one of a 5' cap, a 5' UTR, a 3' UTR and a 3' polyA tail; preferably, the 5' cap is selected from ARCA, mCAP, dmCAP, tmCAP , m7G(5"")ppp(5"")(2""OMeA)pG , m7(3ʺʺOMeG)(5ʺʺ)ppp(5ʺʺ)(2ʺʺOMeA)pG , m7(3""OMeG)(5"")ppp(5"")(2""OMeG)pG , dmCAP or m7G(5"")ppp(5"")(2""OMeG)pG
preferably, the 3' polyA tail has a length of 50 to 200; preferably, the 3' polyA tail has a length of 80 to 200; preferably, the 3' polyA tail has a length of 80 to 120; preferably, the 3' polyA tail has a length of 120;
preferably, the 5'UTR is preferably has a length of 10 to 200 nucleotides, preferably 15 to 100 nucleotides; preferably, the 5'UTR has a nucleotide sequence as set forth in SEQ ID NOs. 16-18; preferably, the 5'UTR has a nucleotide sequence as set forth in SEQ ID NO. 16;
preferably, the 3'UTR has a sequence as set forth in SEQ ID NOs. 19-21; preferably, the 3'UTR has a sequence as set forth in SEQ ID NO. 19;
preferably, one or more uridines in the mRNA are replaced with modified nucleosides; preferably, the modified nucleosides are pseudouridine (ψ), N1-methyl-pseudouridine (m1ψ) or 5-methyl-uridine (m5U); preferably, the modified nucleosides are N1-methyl-pseudouridine (m1ψ).

11. An isolated DNA, wherein the DNA is obtained by reverse transcription of the isolated mRNA as claimed in claim 10, or the DNA is a sequence that can be transcribed into the isolated mRNA as claimed in claim 10.

12. A biological material, comprising any one of an expression cassette, a vector, an engineered bacterium or a cell line, wherein the biological material contains or expresses the isolated mRNA as claimed in claim 10 or the isolated DNA as claimed in claim 11.
